# EUROPEAN PATENT SPECIFICATION

(11) **EP 3 142 689 B1**
(45) Date of publication and mention of the grant of the patent: **11.11.2020**
(21) Application number: 15724454.2
(22) Date of filing: 08.05.2015
(51) Int. Cl.: A61K 39/00, A61K 39/395

(54) **COMBINATION THERAPY FOR TREATING CANCER WITH A POXVIRUS EXPRESSING A TUMOR ANTIGEN AND A MONOCLONAL ANTIBODY AGAINST TIM-3**
KOMBINATIONSTHERAPIE GEGEN KREBS MIT EINEM EIN-KREBSANTIGEN-EXPRIMIERENDEN POXVIRUS UND EINEM MONOKLONALEN ANTIKÖRPER GEGEN TIM-3
THÉRAPIE COMBINÉE POUR LE CANCER AVEC UN POXVIRUS EXPRIMANT UN ANTIGEN TUMORAL ET UN ANTICORPS MONOCLONAL CONTRE TIM-3

(30) Priority: 13.05.2014 US 201461992771 P
(43) Date of publication of application: 22.03.2017
(73) Proprietor: Bavarian Nordic A/S, 3490 Kvistgaard (DK)
(72) Inventor: FOY, Susan, Mountain View, CA 94041 (US); MANDL, Stefanie, San Francisco, CA 94122 (US); ROUNTREE, Ryan, San Jose, CA 95118 (US)
(74) Representative: Bendiksen, Henrik
(86) International application number: PCT/US2015/029885
(87) International publication number: WO 2015/175340

(56) References cited:
- CHAKRABORTY MALA ET AL: "The combined activation of positive costimulatory signals with modulation of a negative costimulatory signal for the enhancement of vaccine-mediated T-cell responses", CANCER IMMUNOLOGY IMMUNOTHERAPY, vol. 56, no. 9, September 2007 (2007-09), pages 1471-1484, XP19514192, ISSN: 0340-7004
- BAGHDADI MUHAMMAD ET AL: "Combined blockade of TIM-3 and TIM-4 augments cancer vaccine efficacy against established melanomas", CANCER IMMUNOLOGY IMMUNOTHERAPY, vol. 62, no. 4, April 2013 (2013-04), pages 629-637, XP002741956, ISSN: 0340-7004
- NGIOW SHIN FOONG ET AL: "Prospects for TIM3-Targeted Antitumor Immunotherapy", CANCER RESEARCH, vol. 71, no. 21, November 2011 (2011-11), pages 6567-6571, XP002741957, ISSN: 0008-5472

## Description

### FIELD OF THE INVENTION

The invention relates to the treatment of cancers using poxviruses encoding a tumor-associated antigen in combination with one or more antagonists of the immune checkpoint molecule TIM-3.

### BACKGROUND OF THE INVENTION

Recombinant poxviruses have been used as vaccines for infectious organism and, more recently, for tumors. Mastrangelo et al. J Clin Invest. 2000; 105 (8): 1031-1034. Two of these poxvirus groups, avipoxvirus and orthopoxvirus have been shown to be effective at battling tumors and involved with potential cancer treatments. Id.

One exemplary avipoxvirus species, fowlpox, has been shown to be a safe vehicle for human administrations as fowlpox virus enters mammalian cells and expresses proteins, but replicates abortively. Skinner et al. Expert Rev Vaccines. 2005 Feb;4(1):63-76. The use of fowlpox virus as a vehicle for expression is being evaluated in numerous clinical trials of vaccines against cancer, malaria, tuberculosis, and AIDS. *Id.*

Vaccinia, the most well-known species of the orthopoxviruses, was used in the world-wide eradication of smallpox and has shown usefulness as a vector and/or vaccine. Recombinant vaccinia vectors have been engineered to express a wide range of inserted genes, including several tumor associated genes such as p97, HER-2/neu, p53 and ETA (Paoletti, et al., 1993).

A useful strain of orthopoxvirus is the Modified Vaccinia Ankara (MVA) virus. MVA was generated by 516 serial passages on chicken embryo fibroblasts of the Ankara strain of vaccinia virus (CVA) (for review see Mayr, A., et al. Infection 3, 6-14 (1975)). As a consequence of these long-term passages, the genome of the resulting MVA virus had about 31 kilobases of its genomic sequence deleted and, therefore, was described as highly host cell restricted for replication to avian cells (Meyer, H. et al., J. Gen. Virol. 72, 1031-1038 (1991)). It was shown in a variety of animal models that the resulting MVA was significantly avirulent (Mayr, A. & Danner, K., Dev. Biol. Stand. 41: 225-34 (1978)). Additionally, this MVA strain has been tested in clinical trials as a vaccine to immunize against the human smallpox disease (Mayr et al., Zbl. Bakt. Hyg. I, Abt. Org. B 167, 375-390 (1987); Stickl et al., Dtsch. med. Wschr. 99, 2386-2392 (1974)). These studies involved over 120,000 humans, including high-risk patients, and proved that, compared to vaccinia-based vaccines, MVA had an improved safety profile with diminished virulence, while maintaining the ability to induce a strong and specific immune response.

In the following decades, MVA was engineered for use as a viral vector for recombinant gene expression or as a recombinant vaccine (Sutter, G. et al., Vaccine 12: 1032-40 (1994)).

Even though Mayr et al. demonstrated during the 1970s that MVA is highly attenuated and avirulent in humans and mammals, certain investigators have reported that MVA is not fully attenuated in mammalian and human cell lines since residual replication might occur in these cells. (Blanchard et al., J Gen Virol 79, 1159-1167 (1998); Carroll & Moss, Virology 238, 198-211 (1997); Altenberger, U.S. Pat. No. 5,185,146; Ambrosini et al., J Neurosci Res 55(5), 569 (1999)). It is assumed that the results reported in these publications have been obtained with various known strains of MVA, since the viruses used essentially differ in their properties, particularly in their growth behavior in various cell lines. Such residual replication is undesirable for various reasons, including safety concerns in connection with use in humans.

Strains of MVA having enhanced safety profiles for the development of safer products, such as vaccines or pharmaceuticals, have been described. *See* International PCT publication WO2002042480 (see also e.g. U.S. Pat. Nos. 6,761,893 and 6,913,752). Such strains are capable of reproductive replication in non-human cells and cell lines, especially in chicken embryo fibroblasts (CEF), but are not capable of significant reproductive replication in certain human cell lines known to permit replication with known vaccinia strains. Such cell lines include a human keratinocyte cell line, HaCat (Boukamp et al. J Cell Biol 106(3): 761-71 (1988)), a human cervix adenocarcinoma cell line, HeLa (ATCC No. CCL-2), a human embryo kidney cell line, 293 (ECACC No. 85120602), and a human bone osteosarcoma cell line, 143B (ECACC No. 91112502). Such strains are also not capable of significant reproductive replication *in vivo,* for example, in certain mouse strains, such as the transgenic mouse model AGR 129, which is severely immune-compromised and highly susceptible to a replicating virus. *See* U.S. Pat. Nos. 6,761,893. One such MVA strain and its derivatives and recombinants, referred to as "MVA-BN," has been described. *See* International PCT publication WO2002042480 (see also e.g. U.S. Pat. Nos. 6,761,893 and 6,913,752) MVA and MVA-BNhave each been engineered for use as a viral vector for recombinant gene expression or as a recombinant vaccine. *See, e.g.,* Sutter, G. et al., Vaccine 12: 1032-40 (1994), International PCT publication WO2002042480 (see also e.g U.S. Pat. Nos. 6,761,893 and 6,913,752).

Certain approaches to cancer immunotherapy have included vaccination with tumor-associated antigens. In certain instances, such approaches have included use of a delivery system to promote host immune responses to tumor-associated antigens. In certain instances, such delivery systems have included recombinant viral vectors. *See, e.g.,* Harrop et al., Front. Biosci. 11:804-817 (2006); Arlen et al., Semin. Oncol. 32:549-555 (2005); Liu et al., Proc. Natl. Acad. Sci. USA 101 (suppl. 2):14567-14571 (2004).

HER-2 is a tumor-associated antigen that is over-expressed in tumor cells of a number of cancer patients. Immunization with various HER-2 polypeptides has been used to generate an immune response against tumor cells expressing this antigen. *See, e.g.,* Renard et al., J. Immunology 171:1588-1595 (2003); Mittendorf et al., Cancer 106:2309-2317 (2006).

An MVA encoding a HER-2 antigen, MVA-BN-HER2, has been shown to exert potent anti-tumor efficacy in a murine model of experimental pulmonary metastasis, despite a strong tumor-mediated immunosuppressive environment characterized by a high frequency of regulatory T cells (T_{reg}) in the lungs. Mandl et al., Cancer Immunol Immunother (2012) 61:19-29. The recombinant MVA was reported to induce strongly Th1-dominated HER-2-specific antibody and T-cell responses. *Id.* The anti-tumor activity was characterized by an increased infiltration of lungs with highly activated, HER-2-specific, CD8+CD11c+ T cells, and was accompanied by a decrease in the frequency of T_{reg} cells in the lung, resulting in a significantly increased ratio of effector T cells to T_{reg} cells. *Id.*

MVA-BN-HER2 has also been shown to be safe and break tolerance to induce specific T and B cell responses in human clinical studies in a metastatic setting. Guardino et al., Cancer Research: December 15, 2009; Volume 69, Issue 24, Supplement 3.

Trastuzumab (Herceptin) is a humanized monoclonal antibody (mAb) targeting the extra-cellular domain of HER2, and has shown clinical efficacy in HER2-positive breast cancer. Wang et al., Cancer Res. 2012 September 1; 72(17): 4417-4428. However, a significant number of patients fail to respond to initial trastuzumab treatment and many trastuzumab-responsive tumors develop resistance after continuous treatment. *Id.*

Inhibitory receptors on immune cells are pivotal regulators of immune escape in cancer. Woo et al., Cancer Res; 72(4); 917-27, 2011. Among these inhibitory receptors, TIM-3 (T-cell immunoglobulin domain and mucin domain-3) is a molecule selectively expressed on a subset of murine IFN-gamma-secreting T helper 1 (Th1) cells and is known to regulate Th1 immunity and tolerance in vivo. Hastings et al. Eur J Immunol. 2009 Sep;39(9):2492-501.

TIM-3 is an immune checkpoint molecule, which has been associated with the inhibition of lymphocyte activity and in some cases induction of lymphocyte anergy. Pardoll D. Nature Reviews 2012 April Vol. 12: 252. TIM-3 is a receptor for galectin 9 (which galectin that is upregrualted in various types of cancers, including breast cancers. *Id.* Anti-TIM-3 antibodies have been shown to promote T cell IFN-γ-mediated antitumor immunity and suppress established tumors. Ngiow et al. (2011) Cancer Res. 71: 3540-3551; Ngiow et al. (2011) Cancer Res. 71: 6567-6571. Baghdadi et al. (2013) Cancer Immunol. Immunother. 62: 629-37 disclosed the administration of a monoclonal antibody against TIM-3 in combination with irradiated B16 melanoma cells engineered to express the flt3 ligand gene, but no combination with viral vaccines was disclosed.

The prior art also includes Chakraborty et al. (2007) Cancer Immunol. Immunother. 56: 1471-84, which discloses vaccines comprising poxvirus vectors expressing a TAA for use in a method of treatment of cancer together with an immune checkpoint blocker that was a monoclonal antibody against CTLA-4, and a vaccinia prime-fowlpox boost for use in a method of cancer treatment. However, no combination with an anti-TIM-3 checkpoint blocker was disclosed.

There is clearly a substantial unmet medical need for additional cancer treatments, including active immunotherapies and cancer vaccines like those described herein.

### BRIEF SUMMARY OF THE INVENTION

The invention encompasses compositions for treating human cancer patients. In one embodiment, the invention includes a combination for use in treating a human cancer patient comprising: (a) a recombinant poxvirus encoding a polypeptide comprising at least one tumor-associated antigen (TAA); and (b) a TIM-3 antagonist that is an antibody, an antisense RNA, or a siRNA.

In one preferred embodiment, the recombinant poxvirus is a recombinant orthopoxvirus or a recombinant avipoxvirus.

In a more preferred embodiment, the recombinant orthopoxivirus is a recombinant vaccinia virus or a recombinant modified Vaccinia Ankara (MVA) virus. In another preferred embodiment, the recombinant orthopoxvirus is MVA-BN.

In another preferred embodiment, the recombinant avipoxvirus is a recombinant fowlpox virus.

In various preferred embodiments, at least one tumor antigen includes, but is not limited to, a CEA, MUC-1, PAP, PSA, HER-2, survivin, tyrosine related protein 1 (tyrp1), tyrosine related protein 2 (tyrp2), or Brachyury antigen.

In other preferred embodiments, the TIM-3 antagonist can include an anti-TIM-3 antibody.

In yet another embodiment, the cancer treatments described herein can be directed against cancers such as, but not limited to, breast cancer, lung cancer, gastric cancer, kidney cancer, liver cancer, melanoma, pancreatic cancer, prostate cancer, ovarian cancer, colorectal cancer, or combinations thereof.

In still another embodiment, the present disclosure additionally encompasses a combination or medicament for use in treating a human cancer patient. The combination or medicament comprises a recombinant poxvirus vector, the poxvirus vector comprising at least one tumor associated antigen (TAA); and a TIM-3 antagonist.

In yet another embodiment, the combination or medicament for use in treating a human cancer patient comprises a recombinant poxvirus encoding a polypeptide comprising at least one tumor-associated antigen (TAA) and a TIM-3 antagonist; and further comprising an antagonist of an immune checkpoint molecule selected from PD-1, LAG-3, CTLA-4 or combinations thereof, wherein the antagonist is an antibody, an antisense RNA or a siRNA.

### BRIEF DESCRIPTION OF THE DRAWINGS

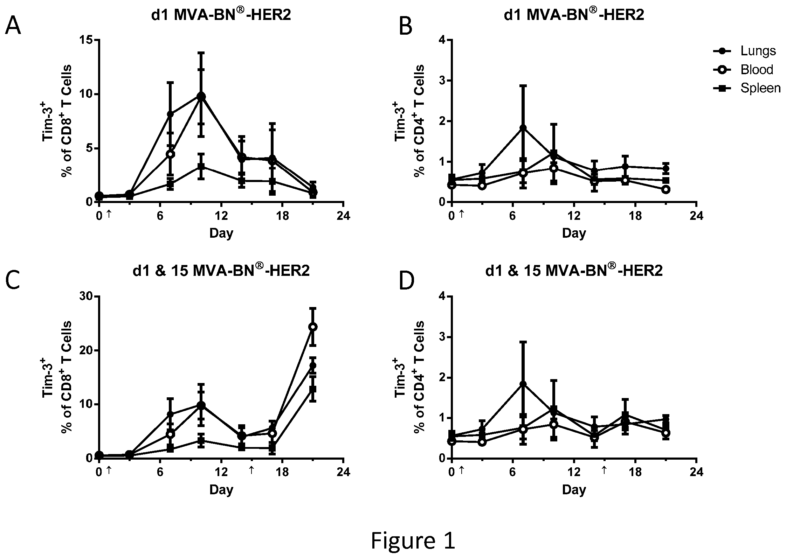

Figure 1. Tim-3 expression increases with MVA-BN-HER2 treatment. Tim-3 expression was measured in mice after day 1 treatment with MVA-BN-HER2 (1E7 Inf.U., t.s.) as described in Example 3. Tim-3 expression after day 1 treatment with MVA-BN-HER2 on CD8 T cells (A) and CD4 T cells (B). Tim-3 expression after day 1 and 15 treatment with MVA-BN-HER2 treatment with MVA-BN-HER2 on CD8 T cells (C) and CD4 T cells (D).

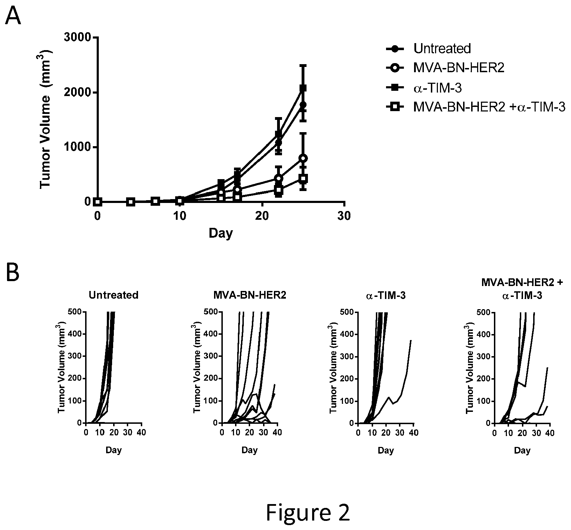

Figure 2. Treatment with MVA-BN-HER2 and Tim-3. Mice were implanted i.d. with CT26-HER-2 tumors on day 1 and treated with MVA-BN-HER2 (1E7 Inf.U. t.s.) and anti-Tim-3 (200 µg, i.p.) on days 1 and 15 as described in Example 4. A) Average tumor volume in mice. B) Individual tumor growth in mice.

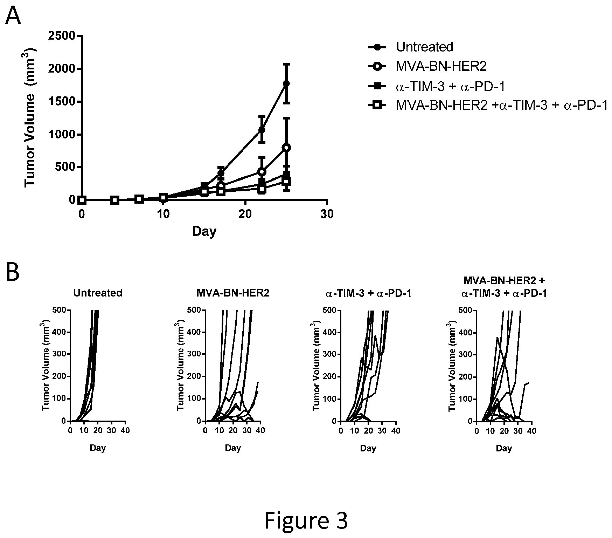

Figure 3. Treatment with MVA-BN-HER2 and Tim-3 and anti-PD-1. Mice were implanted i.d. with CT26-HER-2 tumors on day 1 and treated with MVA-BN-HER2 (1E7 Inf.U. t.s.) and anti-Tim-3 and anti-PD-1 (200 µg each, i.p.) on days 1 and 15 as described in Example 5. A) Average tumor volume in mice. B) Individual tumor growth in mice.

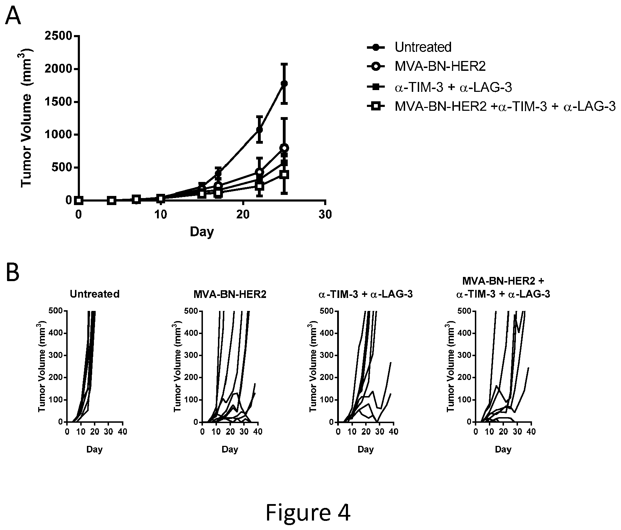

Figure 4. Treatment with MVA-BN-HER2 and anti-Tim-3 and anti-LAG-3. Mice were implanted i.d. with CT26-HER-2 tumors on day 1 and treated with MVA-BN-HER2 (1E7 Inf.U. t.s.) and anti-Tim-3 and anti-LAG-3 (200 µg each, i.p.) on days 1 and 15 as described in Example 6. A) Average tumor volume in mice. B) Individual tumor growth in mice.

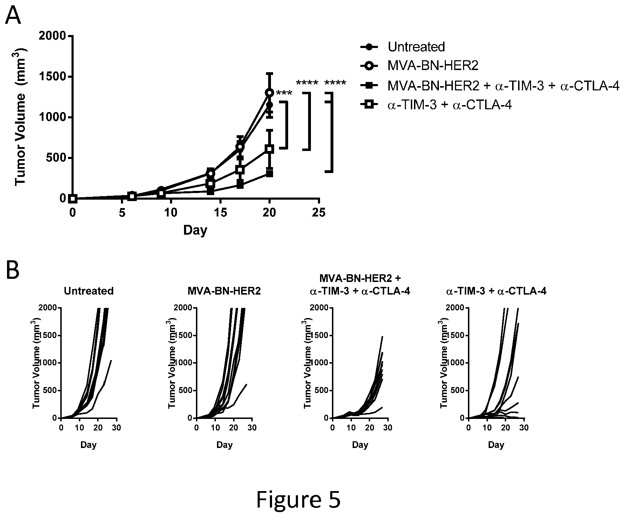

Figure 5. Treatment with MVA-BN-HER2 and anti-Tim-3 and anti-CTLA-4. Mice were implanted i.d. with CT26-HER-2 tumors on day 1 and treated with MVA-BN-HER2 (1E7 Inf.U., s.c. at the tail base) and anti-Tim-3 (200 µg) and anti- CTLA-4 (22 µg) in 100 µL PBS on days 1 and 15 as described in Example 7. A) Average tumor volume in mice. B) Individual tumor growth in mice.

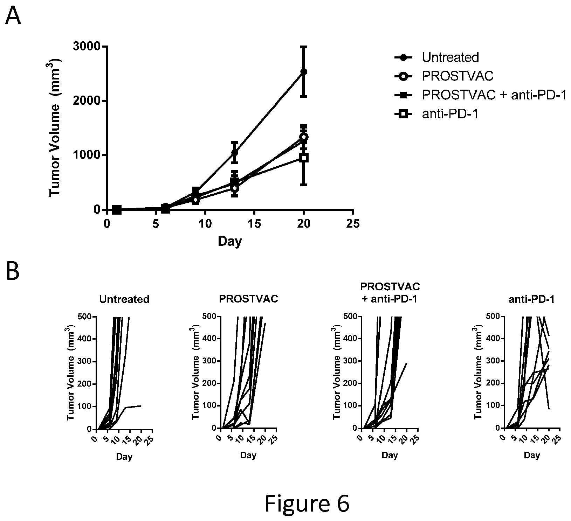

Figure 6. PROSTVAC and anti-PD-1 combination therapy in an E6 solid tumor model as described in Example 9. Mice were treated on day 1 with PROSTVAC-V, and days 8 and 15 with PROSTVAC-F. Anti-PD-1 was given on days 1 and 15. A) Average tumor volume in mice. B) Individual tumor growth in mice.

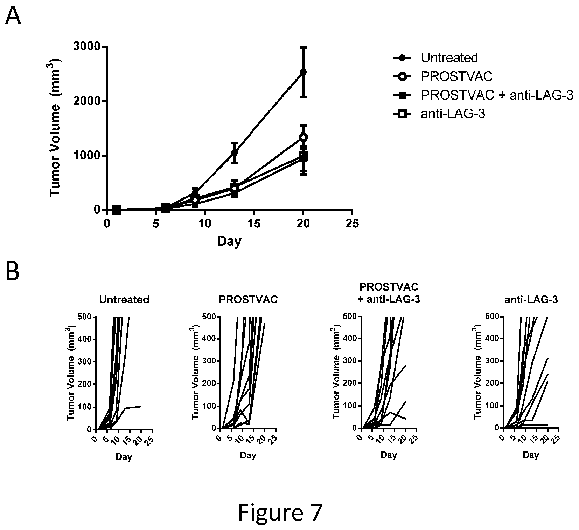

Figure 7. PROSTVAC and anti-LAG-3 combination therapy in an E6 solid tumor model. Mice were treated on day 1 with PROSTVAC-V and days 8 and 15 with PROSTVAC-F as described in Example 10. Anti-LAG-3 was given on days 1 and 15. A) Average tumor volume in mice. B) Individual tumor growth in mice.

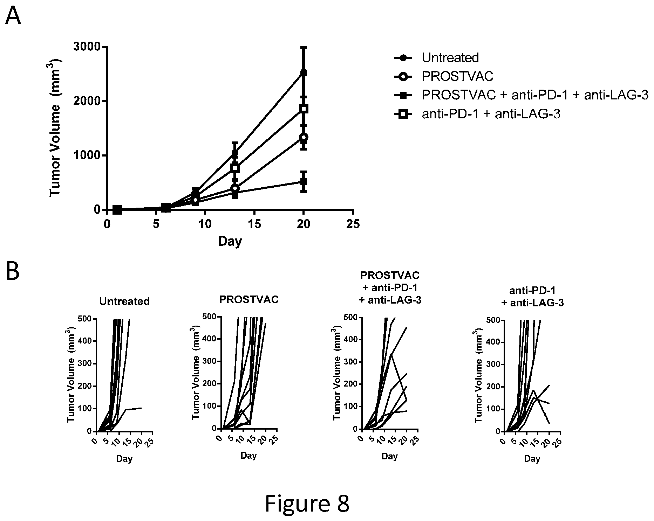

Figure 8. PROSTVAC in combination with anti-PD-1 and anti-LAG-3 in an E6 solid tumor model. Mice were treated on day 1 with PROSTVAC-V and days 8 and 15 with PROSTVAC-F as described in Example 11. Anti-PD-1 and anti-LAG-3 were given on days 1 and 15. A) Average tumor volume in mice. B) Individual tumor growth in mice.

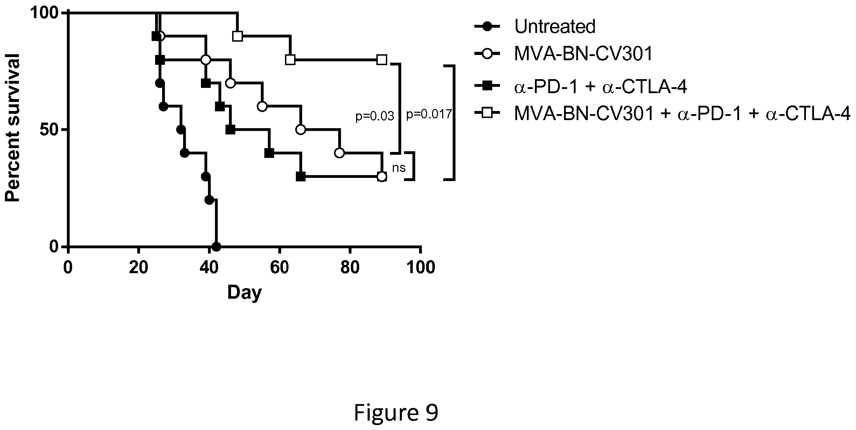

Figure 9. Overall survival in mice treated with MVA-BN-CV301 and anti-PD-1 and anti-CTLA-4. Female C57/BL6 mice (6-8 weeks old, ∼ 20 g, Simonsen Laboratories, Gilroy,CA) were implanted on day 1 i.v. with 1.0×10^6 MC38-MUC1 cells in 300 µL DPBS as described in Example 12. Mice were treated with MVA-BN-CV301 (4E5 Inf.U. subcutaneously, s.c. above the tail base) and treated with anti-CTLA-4 and anti-PD-1 (200 µg each) i.p. on days 4 and 18.

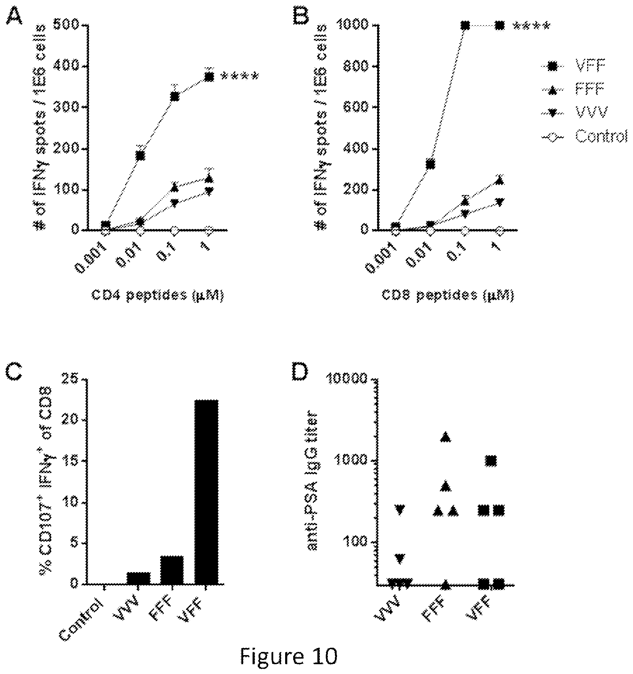

Figure 10. Mice were treated as described in Example 13. Pooled splenocytes were assayed for PSA-specific responses by IFN□ ELISPOT (A, B) and cytotoxic activity by flow cytometry (% CD107⁺ IFNγ⁺ CD8 T cells) (C). Anti-PSA IgG titers were determined by ELISA for each individual mouse (D). For ELISPOT, Graphs show representative data of four independently performed experiments.

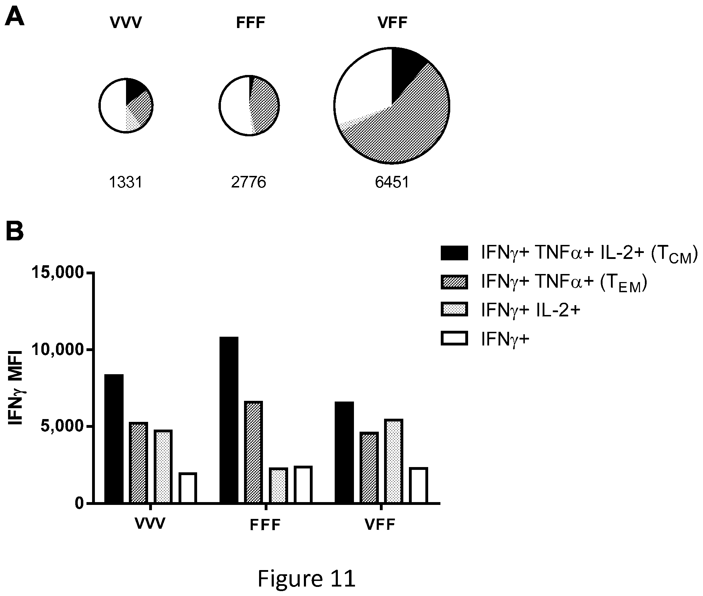

Figure 11. Mice were treated as described in Example 14. (A) The pie charts are weighted in size to reflect the numbers of detected cells (total numbers of PSA-specific CD8 per million T cells are indicated below each chart). (B) Amount of IFNγ production on a per cell basis as measured by mean fluorescence intensity (MFI). Graphs show representative data of two independently performed experiments.

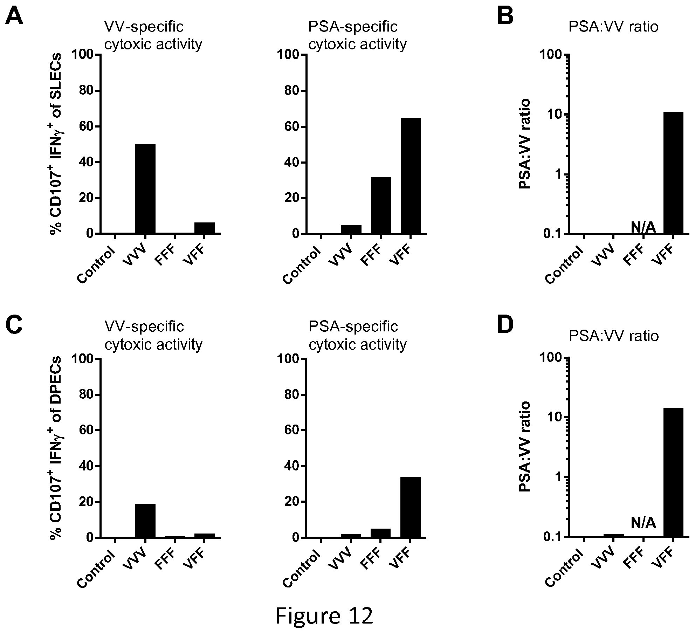

Figure 12. Mice were treated as described in Example 15. Pooled splenocytes were assayed for vaccinia virus (VV)-specific (A and C panels on left) or PSA-specific (A and C panels on right) cytotoxic activity by flow cytometry (% CD107+ IFNγ+ CD8 T cells) 14 days after the last treatment. Graphs show representative data of two independently performed experiments.

### DETAILED DESCRIPTION OF THE INVENTION

A number of current clinical trial involve therapies employ vaccinia-, Modified Vaccinia Ankara (MVA)-, and fowlpox-based vectors that were engineered to express one or more tumor-associated antigens (TAA). These vectors are used alone or in prime-boost strategies to generate an active immune response against a variety of cancers. PROSTVAC® employs a prime-boost strategy using vaccinia and fowlpox expressing PSA and TRICOM™ and is currently in a global Phase III clinical trial (PROSPECT) for castration-resistant metastatic prostate cancer. CV301, or CV-301, employs a heterologous prime-boost strategy using vaccinia and fowlpox expressing MUC-1 antigen, CEA, and TRICOM™ and is currently in a Phase II clinical trial for Bladder Cancer.

MVA-BN-HER2 (Mandl et al, 2012), is in Phase I clinical trials for the treatment of HER-2⁺-breast cancer. This recombinant vector is derived from the highly attenuated Modified Vaccinia Ankara (MVA) virus stock known as MVA-BN. It expresses a modified form of HER-2 (designated HER2) consisting of the extracellular domain of HER-2 that has been engineered to include two universal T cell epitopes from tetanus toxin (TTp2 and TTp30) to facilitate the induction of effective immune responses against HER-2.

To further enhance the anti-tumor efficacy of the poxvirus-based immunotherapy, MVA-BN-HER2 was combined with a monoclonal antibody that blocks the activity of TIM-3, an immune checkpoint protein that down-regulates T cell activation. In the CT26-HER-2 tumor model, tumor volumes decreased significantly as compared to tumors treated with an anti-TIM-3 antibody alone and MVA-BN-HER2 alone.

To further review the ability of TIM-3 antagonists to treat cancer patients in combination with poxviruses, MVA-BN-HER2 and anti-TIM-3 antibodies were tested in combination with additional immune checkpoint antagonists and agonists. MVA-BN-HER2 and an anti-TIM-3 antibody in combination with an anti-PD-1 antibody resulted in a decrease in tumor volume, as did MVA-BN-HER2 and an anti-TIM-3 antibody in combination with an anti-LAG-3 antibody. Further, MVA-BN-HER2 and an anti-TIM-3 antibody in combination with an anti-CTLA-4 antibody resulted in a decrease in tumor volume.

PROSTVAC® and MVA-BN CV-301 were each also tested in combination with various antagonist antibodies directed against PD-1 and LAG-3 in various tumor models. Combinations were found to enhance the effects of PROSTVAC® and MVA-BN CV301.

### Poxvirus Encoding a Polypeptide Comprising a Tumor Antigen

In one embodiment of the invention, there is a combination for use in treating a human cancer patient comprising a recombinant poxvirus encoding and/or expressing a polypeptide comprising at least one tumor antigen or tumor associated antigen; and at least one TIM-3 antagonist that is an antibody, an antisense RNA, or a siRNA.

In one embodiment, the recombinant poxvirus expressing a tumor antigen is preferably an orthopoxvirus such as, but not limited to, a vaccinia virus, a Modified Vaccinia Ankara (MVA) virus, or MVA-BN.

Examples of vaccinia virus strains are the strains Temple of Heaven, Copenhagen, Paris, Budapest, Dairen, Gam, MRIVP, Per, Tashkent, TBK, Tom, Bern, Patwadangar, BIEM, B-15, Lister, EM-63, New York City Board of Health, Elstree, Ikeda and WR. A preferred vaccinia virus (VV) strain is the Wyeth (DRYVAX) strain (U.S. Patent 7,410,644). Another preferred VV strain is a modified vaccinia virus Ankara (MVA) (Sutter, G. et al. [1994], Vaccine 12: 1032-40). Another preferred VV strain is MVA-BN.

Examples of MVA virus strains that are useful in the practice of the present invention and that have been deposited in compliance with the requirements of the Budapest Treaty are strains MVA 572, deposited at the European Collection of Animal Cell Cultures (ECACC), Vaccine Research and Production Laboratory, Public Health Laboratory Service, Centre for Applied Microbiology and Research, Porton Down, Salisbury, Wiltshire SP4 0JG, United Kingdom, with the deposition number ECACC 94012707 on January 27, 1994, and MVA 575, deposited under ECACC 00120707 on December 7, 2000. MVA-BN, deposited on Aug. 30, 2000 at the European Collection of Cell Cultures (ECACC) under number V00083008, and its derivatives, are additional exemplary strains.

Although MVA-BN is preferred for its higher safety (less replication competent), all MVAs are suitable for this invention. According to an embodiment of the present invention, the MVA strain is MVA-BN and its derivatives. A definition of MVA-BN and its derivatives is given in PCT/EP01/13628.

In one embodiment, the invention encompasses the use of recombinant orthopoxviruses, preferably a vaccinia virus (VV), Wyeth strain VV, ACAM 1000, ACAM 2000, an MVA, or an MVA-BN viruses, for cancer therapy. Recombinant orthopoxviruses can be generated by insertion of heterologous sequences into an orthopoxvirus.

In another embodiment the invention encompasses the use of a recombinant avipox virus, preferably a fowlpox virus. Recombinant avipoxvirues can be generated by insertion of heterologous sequences into an avipoxvirus.

In certain embodiments, the orthopoxvirus comprises at least one tumor-associated antigen (TAA). In a preferred embodiment, the TAA includes, but is not limited to, a CEA, MUC-1, PAP, PSA, HER-2, survivin, tyrp1, tyrp2, or Brachyury antigen.

In further embodiments, the tumor-associated antigen is modified to include one or more foreign T_{H} epitopes. Such a cancer immunotherapeutic agent is described herein in a non-limiting example and is referred to as "MVA-BN-mHER2." As described herein, such cancer immunotherapeutic agents, including, but not limited to MVA-BN-mHER2, are useful for the treatment of cancer. The invention allows for the use of such agents in prime/boost vaccination regimens of humans and other mammals, including immunocompromised patients; and inducing both humoral and cellular immune responses, such as inducing a Th1 immune response in a pre-existing Th2 environment.

In certain embodiments, the MVA is MVA-BN, deposited on Aug. 30, 2000, at the European Collection of Cell Cultures (ECACC) under number V00083008, and described in International PCT publication WO2002042480 (see also e.g U.S. Pat. Nos. 6,761,893 and 6,913,752). As described in those patent publications, MVA-BN does not reproductively replicate in cell lines 293, 143B, HeLa and HaCat. In particular, MVA-BN exhibits an amplification ratio of 0.05 to 0.2 in the human embryo kidney cell line 293. In the human bone osteosarcoma cell line 143B, MVA-BN exhibits an amplification ratio of 0.0 to 0.6. MVA-BN exhibits an amplification ratio of 0.04 to 0.8 in the human cervix adenocarcinoma cell line HeLa, and 0.02 to 0.8 in the human keratinocyte cell line HaCat. MVA-BN has an amplification ratio of 0.01 to 0.06 in African green monkey kidney cells (CV1: ATCC No. CCL-70).

The amplification ratio of MVA-BN is above 1 in chicken embryo fibroblasts (CEF: primary cultures) as described in International PCT publication WO2002042480 (see also e.g. U.S. Pat. Nos. 6,761,893 and 6,913,752). The virus can be easily propagated and amplified in CEF primary cultures with a ratio above 500.

In certain embodiments, a recombinant MVA is a derivative of MVA-BN. Such "derivatives" include viruses exhibiting essentially the same replication characteristics as the deposited strain (ECACC No. V00083008), but exhibiting differences in one or more parts of its genome. Viruses having the same "replication characteristics" as the deposited virus are viruses that replicate with similar amplification ratios as the deposited strain in CEF cells and the cell lines, HeLa, HaCat and 143B; and that show similar replication characteristics *in vivo,* as determined, for example, in the AGR129 transgenic mouse model.

In certain embodiments, the poxvirus is a recombinant vaccinia virus that contains additional nucleotide sequences that are heterologous to the poxvirus. In certain such embodiments, the heterologous sequences code for epitopes that induce a response by the immune system. Thus, in certain embodiments, the recombinant poxvirus is intended to vaccinate against the proteins or agents comprising the epitope. In a preferred embodiment, the epitope is a tumor-associated antigen, preferably, HER-2. In one embodiment, the HER-2 antigen comprises the sequence of SEQ ID NO:2.

In other preferred embodiments, the epitope is a tumor-associated antigen selected from an antigen such as, but not limited to, CEA, MUC-1, PAP, PSA, HER-2, survivin, tyrp1, tyrp2, or Brachyury.

In certain embodiments, a heterologous nucleic acid sequence encoding a tumor-associated antigen described herein is inserted into a non-essential region of the virus genome. In certain of those embodiments, the heterologous nucleic acid sequence is inserted at a naturally occurring deletion site of the MVA genome as described in PCT/EP96/02926. Methods for inserting heterologous sequences into the poxviral genome are known to a person skilled in the art.

In another embodiment, the recombinant poxvirus expressing a tumor antigen is preferably an avipoxvirus, such as but not limited to a fowlpox virus.

In other embodiments, the recombinant poxvirus expressing a tumor antigen is a combination of a vaccinia virus expressing a tumor antigen and an avipoxvirus, such as fowlpox, expressing a tumor antigen.

The term "avipoxvirus" refers to any avipoxvirus, such as Fowlpoxvirus, Canarypoxvirus, Uncopoxvirus, Mynahpoxvirus, Pigeonpoxvirus, Psittacinepoxvirus, Quailpoxvirus, Peacockpoxvirus, Penguinpoxvirus, Sparrowpoxvirus, Starlingpoxvirus and Turkeypoxvirus. Preferred avipoxviruses are Canarypoxvirus and Fowlpoxvirus.

An example of a canarypox virus is strain Rentschler. A plaque purified Canarypox strain termed ALVAC (U.S. Pat. No. 5,766,598) was deposited under the terms of the Budapest treaty with the American Type Culture Collection (ATCC), accession number VR-2547. Another Canarypox strain is the commercial canarypox vaccine strain designated LF2 CEP 524 24 10 75, available from Institute Merieux, Inc.

Examples of a Fowlpox virus are strains FP-1, FP-5, TROVAC (U.S. Pat. No. 5,766,598), and POXVAC-TC (U.S. Patent 7,410,644). FP-1 is a Duvette strain modified to be used as a vaccine in one-day old chickens. The strain is a commercial fowlpox virus vaccine strain designated O DCEP 25/CEP67/2309 October 1980 and is available from Institute Merieux, Inc. FP-5 is a commercial fowlpox virus vaccine strain of chicken embryo origin available from American Scientific Laboratories (Division of Schering Corp.) Madison, Wis., United States Veterinary License No. 165, serial No. 30321.

Examples of vaccinia virus strains are the strains Temple of Heaven, Copenhagen, Paris, Budapest, Dairen, Gam, MRIVP, Per, Tashkent, TBK, Tom, Bern, Patwadangar, BIEM, B-15, Lister, EM-63, New York City Board of Health, Elstree, Ikeda and WR. A preferred vaccinia virus (VV) strain is the Wyeth (DRYVAX) strain (U.S. Patent 7,410,644). Another preferred VV strain is a modified vaccinia virus Ankara (MVA) (Sutter, G. et al. [1994], Vaccine 12: 1032-40). Another preferred VV strain is MVA-BN.

In certain embodiments, the avipox virus includes at least one tumor-associated antigen (TAA). In a preferred embodiment, the TAA includes, but is not limited to, a CEA, MUC-1, PAP, PSA, HER-2, survivin, tyrp1, tyrp2, or Brachyury antigen.

In other embodiments, the recombinant poxvirus expressing a tumor antigen is a combination of a vaccinia virus expressing a tumor antigen and an avipoxvirus, such as fowlpox, expressing a tumor antigen. It is contemplated that the vaccinia virus and fowlpox virus combination can be administered as a heterologous prime-boost regimen. In one non-limiting example, the heterologous prime-boost regimen is PROSTVAC® or CV301.

For the preparation of vaccines, the poxvirus can be converted into a physiologically acceptable form. In certain embodiments, such preparation is based on experience in the preparation of poxvirus vaccines used for vaccination against smallpox, as described, for example, in Stickl, H. et al., Dtsch. med. Wschr. 99, 2386-2392 (1974).

An exemplary preparation follows. Purified virus is stored at -80°C with a titer of 5 x 10⁸ TCID₅₀/ml formulated in 10 mM Tris, 140 mM NaCl, pH 7.4. For the preparation of vaccine shots, e.g., 10²-10⁸ particles of the virus can be lyophilized in phosphate-buffered saline (PBS) in the presence of 2% peptone and 1% human albumin in an ampoule, preferably a glass ampoule. Alternatively, the vaccine shots can be prepared by stepwise, freeze-drying of the virus in a formulation. In certain embodiments, the formulation contains additional additives such as mannitol, dextran, sugar, glycine, lactose, polyvinylpyrrolidone, or other additives, such as, including, but not limited to, antioxidants or inert gas, stabilizers or recombinant proteins (e.g. human serum albumin) suitable for *in vivo* administration. The ampoule is then sealed and can be stored at a suitable temperature, for example, between 4°C and room temperature for several months. However, as long as no need exists, the ampoule is stored preferably at temperatures below -20°C.

In various embodiments involving vaccination or therapy, the lyophilisate is dissolved in 0.1 to 0.5 ml of an aqueous solution, preferably physiological saline or Tris buffer, and administered either systemically or locally, i.e., by parenteral, subcutaneous, intravenous, intramuscular, intranasal, intradermal, or any other path of administration known to a skilled practitioner. Optimization of the mode of administration, dose, and number of administrations is within the skill and knowledge of one skilled in the art.

In certain embodiments, attenuated vaccinia virus strains are useful to induce immune responses in immune-compromised animals, e.g., monkeys (CD4<400/µl of blood) infected with SIV, or immune-compromised humans. The term "immune-compromised" describes the status of the immune system of an individual that exhibits only incomplete immune responses or has a reduced efficiency in the defense against infectious agents.

### Certain Exemplary Tumor-Associated Antigens

In certain embodiments, an immune response is produced in a subject against a cell-associated polypeptide antigen. In certain such embodiments, a cell-associated polypeptide antigen is a tumor-associated antigen.

The term "polypeptide" refers to a polymer of two or more amino acids joined to each other by peptide bonds or modified peptide bonds. The amino acids may be naturally occurring as well as non-naturally occurring, or a chemical analogue of a naturally occurring amino acid. The term also refers to proteins, i.e. functional biomolecules comprising at least one polypeptide; when comprising at least two polypeptides, these may form complexes, be covalently linked, or may be non-covalently linked. The polypeptide(s) in a protein can be glycosylated and/or lipidated and/or comprise prosthetic groups.

Preferably, the tumor-associated antigen includes, but is not limited to, CEA, MUC-1, PAP, PSA, HER-2, survivin, tyrosine related protein 1 (tyrp1), tyrosine related protein 2 (tyrp2), Brachyury alone or in combinations. Such exemplary combination may include CEA and MUC-1, also known as CV301. Other exemplary combinations may include PAP and PSA.

Numerous tumor-associated antigens are known in the art. Exemplary tumor-associated antigens include, but are not limited to, 5 alpha reductase, alpha-fetoprotein, AM-1, APC, April, BAGE, beta-catenin, Bcl12, bcr-abl, CA-125, CASP-8/FLICE, Cathepsins, CD19, CD20, CD21, CD23, CD22, CD33 CD35, CD44, CD45, CD46, CD5, CD52, CD55, CD59, CDC27, CDK4, CEA, c-myc, Cox-2, DCC, DcR3, E6/E7, CGFR, EMBP, Dna78, farnesyl transferase, FGF8b, FGF8a, FLK-1/KDR, folic acid receptor, G250, GAGE-family, gastrin 17, gastrin-releasing hormone, GD2/GD3/GM2, GnRH, GnTV, GP1, gp100/Pmel17, gp-100-in4, gp15, gp75/TRP-1, hCG, heparanse, Her2/neu, HMTV, Hsp70, hTERT, IGFR1, IL-13R, iNOS, Ki67, KIAA0205, K-ras, H-ras, N-ras, KSA, LKLR-FUT, MAGE-family, mammaglobin, MAP17, melan-A/MART-1, mesothelin, MIC A/B, MT-MMPs, mucin, NY-ESO-1, osteonectin, p15, P170/MDR1, p53, p97/melanotransferrin, PAI-1, PDGF, uPA, PRAME, probasin, progenipoientin, PSA, PSM, RAGE-1, Rb, RCAS1, SART-1, SSX-family, STAT3, STn, TAG-72, TGF-alpha, TGF-beta, Thymosin-beta-15, TNF-alpha, TP1, TRP-2, tyrosinase, VEGF, ZAG, p16INK4, and glutathione-S-transferase.

A preferred PSA antigen comprises the amino acid change of isoleucine to leucine at position 155.

One exemplary tumor-associated antigen is HER-2. HER-2 is a member of the epidermal growth factor receptor family (c-erbB) which consists of four different receptors to date: c-erbB-1 (EGFr), c-erbB-2 (HER-2, c-Neu), c-erbB-3 and c-erbB-4 (Salomon et al, 1995). C-erbB-3 and c-erbB-4 are less well characterized than EGFr and HER-2. HER-2 is an integral membrane glycoprotein. The mature protein has a molecular weight of 185 kD with structural features that closely resemble the EGFr receptor (Prigent et al, 1992). EGFr is also an integral membrane receptor consisting of one subunit. It has an apparent molecular weight of 170 kD and consists of a surface ligand-binding domain of 621 amino acids, a single hydrophobic transmembrane domain of 23 amino acids, and a highly conserved cytoplasmic tyrosine kinase domain of 542 amino acids. The protein is N-glycosylated (Prigent et al, 1994).

All proteins in this family are tyrosine kinases. Interaction with the ligand leads to receptor dimerization, which increases the catalytic action of the tyrosine kinase (Bernard. 1995, Chantry 1995). The proteins within the family are able to homo- and heterodimerise, which is important for their activity. The EGFr conveys growth promoting effects and stimulates uptake of glucose and amino acids by cells (Prigent et al 1992). HER-2 also conveys growth promoting signals.

The epidermal growth factor receptor is expressed on normal tissues in low amounts, but it is overexpressed in many types of cancers. EGFr is overexpressed in breast cancers (Earp et al, 1993, Eppenberger 1994), gliomas (Schlegel et al, 1994), gastric cancer (Tkunaga et al, 1995), cutaneous squamous carcinoma (Fujii 1995), ovarian cancer (van Dam et al, 1994) and others. HER-2 is also expressed on few normal human tissues in low amount, most characteristically on secretory epithelia. Over-expression of HER-2 occurs in about 30% of breast, gastric, pancreatic, bladder and ovarian cancers.

The expression of these receptors varies depending on the degree of differentiation of the tumors and the cancer type, e.g., in breast cancer, primary tumors overexpress both receptors; whereas in gastric cancer, the overexpression occurs at a later stage in metastatic tumours (Salomon et al, 1995). The number of overexpressed receptors on carcinoma cells is greater than 10⁶/cell for several head and neck cancers, vulva, breast and ovarian cancer lines isolated from patients (Dean et al, 1994).

There are several reasons why the EGFr family of receptors constitutes suitable targets for tumor immunotherapy. First, they are overexpressed in many types of cancers, which should direct the immune response towards the tumor. Second, the tumors often express or overexpress the ligands for this family of receptors and some are hypersensitive to the proliferative effects mediated by the ligands. Third, patients with tumors that overexpress growth factor receptors often have a poor prognosis. The overexpression has been closely linked with poor prognosis especially in breast cancer, lung cancer, and bladder cancer and can be associated with invasive/metastatic phenotypes, which are rather insensitive to conventional therapies (Eccles et al, 1994).

### Modified Tumor-Associated Antigens

In certain embodiments, a cell-associated polypeptide antigen is modified such that a CTL response is induced against a cell which presents epitopes derived from a polypeptide antigen on its surface, when presented in association with an MHC Class I molecule on the surface of an APC. In certain such embodiments, at least one first foreign TH epitope, when presented, is associated with an MHC Class II molecule on the surface of the APC. In certain such embodiments, a cell-associated antigen is a tumor-associated antigen.

Exemplary APCs capable of presenting epitopes include dendritic cells and macrophages. Additional exemplary APCs include any pino- or phagocytizing APC, which is capable of simultaneously presenting 1) CTL epitopes bound to MHC class I molecules and 2) T_{H} epitopes bound to MHC class II molecules.

In certain embodiments, modifications to one or more of the tumor-associated antigens (TAAs) presented herein, such as, but not limited to, CEA, MUC-1, PAP, PSA, HER-2, survivin, tyrp1, tyrp2, or Brachyury are made such that, after administration to a subject, polyclonal antibodies are elicited that predominantly react with the one or more of the TAAs described herein . Such antibodies could attack and eliminate tumor cells as well as prevent metastatic cells from developing into metastases. The effector mechanism of this anti-tumor effect would be mediated via complement and antibody dependent cellular cytotoxicity. In addition, the induced antibodies could also inhibit cancer cell growth through inhibition of growth factor dependent oligo-dimerisation and internalization of the receptors. In certain embodiments, such modified TAAs polypeptide antigens could induce CTL responses directed against known and/or predicted TAA epitopes displayed by the tumor cells.

In certain embodiments, a modified TAA polypeptide antigen comprises a CTL epitope of the cell-associated polypeptide antigen and a variation, wherein the variation comprises at least one CTL epitope of a foreign T_{H} epitope. Certain such modified TAAs can include in one non-limiting example one or more HER-2 polypeptide antigens comprising at least one CTL epitope and a variation comprising at least one CTL epitope of a foreign T_{H} epitope, and methods of producing the same, are described in U.S. Patent No. 7,005,498 and U.S. Patent Pub. Nos. 2004/0141958 and 2006/0008465.

In certain embodiments, a foreign T_{H} epitope is a naturally-occurring "promiscuous" T-cell epitope. Such promiscuous T-cell epitopes are active in a large proportion of individuals of an animal species or an animal population. In certain embodiments, a vaccine comprises such promiscuous T-cell epitopes. In certain such embodiments, use of promiscuous T-cell epitopes reduces the need for a very large number of different CTL epitopes in the same vaccine. Exemplary promiscuous T-cell epitopes include, but are not limited to, epitopes from tetanus toxin, including but not limited to, the P2 and P30 epitopes (Panina-Bordignon et al., 1989), diphtheria toxin, Influenza virus hemagluttinin (HA), and P. falciparum CS antigen.

Additional promiscuous T-cell epitopes include peptides capable of binding a large proportion of HLA-DR molecules encoded by the different HLA-DR. See, e.g., WO 98/23635 (Frazer IH et al., assigned to The University of Queensland); Southwood S et. al, 1998, J. Immunol. 160: 3363 3373; Sinigaglia F et al., 1988, Nature 336: 778 780; Rammensee HG et al., 1995, Immunogenetics 41: 4 178 228; Chicz RM et al., 1993, J. Exp. Med 178: 27 47; Hammer J et al., 1993, Cell 74: 197 203; and Falk K et al., 1994, Immunogenetics 39: 230 242. The latter reference also deals with HLA-DQ and -DP ligands. All epitopes listed in these references are relevant as candidate natural epitopes as described herein, as are epitopes which share common motifs with these.

In certain other embodiments, the promiscuous T-cell epitope is an artificial T-cell epitope which is capable of binding a large proportion of haplotypes. In certain such embodiments, the artificial T-cell epitope is a pan DR epitope peptide ("PADRE") as described in WO 95/07707 and in the corresponding paper Alexander J et al., 1994, Immunity 1: 751 761.

### mHER2

Various modified HER-2 polypeptide antigens and methods for producing the same are described in U.S. Patent No. 7,005,498 and U.S. Patent Pub. Nos. 2004/0141958 and 2006/0008465. Those documents describe various modified HER-2 polypeptide antigens comprising promiscuous T-cell epitopes at different positions in the HER-2 polypeptide.

The human HER-2 sequence can be divided into a number of domains based solely on the primary structure of the protein. Those domains are as follows. The extracellular (receptor) domain extends from amino acids 1-654 and contains several subdomains as follows: Domain I (N-terminal domain of mature polypeptide) extends from amino acids 1-173; Domain II (Cysteine rich domain, 24 cysteine residues) extends from amino acids 174-323; Domain III (ligand binding domain in the homologous EGF receptor) extends from amino acids 324-483; and Domain IV (Cysteine rich domain, 20 cysteine residues) extends from amino acids 484-623. The transmembrane residues extend from amino acids 654-675. The intracellular (Kinase) domain extends from amino acids 655-1235 and contains the tyrosine kinase domain, which extends from amino acids 655-1010 (core TK domain extends from 725-992); and the C-terminal domain, which extends from amino acids 1011-1235.

Selection of sites in the amino acid sequence of HER-2 to be displaced by either the P2 or P30 human T helper epitopes is described in U.S. Patent No. 7,005,498 and U.S. Patent Pub. Nos. 2004/0141958 and 2006/0008465. To summarize, the following parameters were considered:
1. Known and predicted CTL epitopes;
2. Homology to related receptors (EGFR in particular);
3. Conservation of cysteine residues;
4. Predicted loop, α-helix and β-sheet structures;
5. Potential N-glycosylation sites;
6. Prediction of exposed and buried amino acid residues;
7. Domain organization.

The CTL epitopes appear to be clustered in domain I, domain III, the TM domain and in two or three "hot spots" in the TK domain. As described in U.S. Patent No. 7,005,498 and U.S. Patent Pub. Nos. 2004/0141958 and 2006/0008465, these should be largely conserved.

Regions with a high degree of homology with other receptors are likely to be structurally important for the "overall" tertiary structure of HER-2, and hence for antibody recognition, whereas regions with low homology possibly can be exchanged with only local alterations of the structure as the consequence.

Cysteine residues are often involved in intramolecular disulphide bridge formation and are thus involved in the tertiary structure and should not be changed. Regions predicted to form alpha-helix or beta-sheet structures should be avoided as insertion points of foreign epitopes, as these regions are thought to be involved in folding of the protein.

Potential N-glycosylation sites should be conserved if mannosylation of the protein is desired.

Regions predicted (by their hydrophobic properties) to be interior in the molecule preferably should be conserved as these could be involved in the folding. In contrast, solvent exposed regions could serve as candidate positions for insertion of the model T_{H} epitopes P2 and P30.

Finally, the domain organization of the protein should be taken into consideration because of its relevance for protein structure and function.

As described in U.S. Patent No. 7,005,498 and U.S. Patent Pub. Nos. 2004/0141958 and 2006/0008465, the focus of the strategy has been to conserve the structure of the extracellular part of HER-2 as much as possible, because this is the part of the protein which is relevant as a target for neutralizing antibodies. By contrast, the intracellular part of native membrane bound HER-2 on the surface of cancer cells is inaccessible for the humoral immune system.

Various exemplary constructs using the P2 and P30 epitopes of tetanus toxin inserted in various domains of HER-2 are provided in U.S. Patent No. 7,005,498 and U.S. Patent Pub. Nos. 2004/0141958 and 2006/0008465. One exemplary modified HER-2 polypeptide antigen, referred to as "mHER2," comprises the extracellular domains and nine amino acids of the transmembrane domain; the P2 epitope inserted in Domain II between amino acid residues 273 to 287 of the modified HER-2 polypeptide; and the P30 epitope inserted in Domain IV between amino acid residues 655 to 675 of the modified HER-2 polypeptide.

### Recombinant MVA-BN-mHER2

In a non-limiting embodiment, recombinant MVA comprising a tumor-associated antigen, e.g., MVA-BN-mHER2, is constructed as follows. The initial virus stock is generated by recombination in cell culture using a cell type permissive for replication, e.g., CEF cells. Cells are both inoculated with an attenuated vaccinia virus, e.g., MVA-BN, and transfected with a recombination plasmid (e.g., pBN146) that encodes the tumor-associated antigen, e.g., HER2, sequence and flanking regions of the virus genome. In one non-limiting embodiment, the plasmid pBN146 contains sequences which are also present in MVA-BN (the 14L and 15L open reading frames). The HER2 sequence is inserted between the MVA-BN sequences to allow for recombination into the MVA-BN viral genome. In certain embodiments, the plasmid also contains a selection cassette comprising one or more selection genes to allow for selection of recombinant constructs in CEF cells. In a preferred embodiment, the recombinant MVA encodes a polypeptide comprising SEQ ID NO:2.

Simultaneous infection and transfection of cultures allows homologous recombination to occur between the viral genome and the recombination plasmid. Insert-carrying virus is then isolated, characterized, and virus stocks prepared. In certain embodiments, virus is passaged in CEF cell cultures in the absence of selection to allow for loss of the region encoding the selection genes, gpt and EGFP.

### Antagonists of TIM-3, PD-1, PDL-1, LAG-3, and CTLA-4

At least in one aspect, the invention encompasses antagonists of T-cell immunoglobulin and mucin domain 3 (TIM-3), Programmed Cell Death Protein 1 (PD-1), Programmed Death-Ligand 1 (PDL-1), Lymphocyte-activation gene 3 (LAG-3), and Cytotoxic T-Lymphocyte Antigen 4(CTLA-4). An antagonist of TIM-3, PD-1, PDL-1, LAG-3, or CTLA-4 interferes with TIM-3, PD-1, PDL-1, LAG-3, or CTLA-4 function, respectively, and is an antibody, an antisense RNA, or a siRNA.

Such antagonists of TIM-3, PD-1, PDL-1, LAG-3, and CTLA-4 can include antibodies which specifically bind to TIM-3, PD-1, PDL-1, LAG-3, and CTLA-4 and inhibit and/or block TIM-3, PD-1, PDL-1, LAG-3, and CTLA-4 biological activity and function, respectively.

Other antagonists of TIM-3, PD-1, PDL-1, LAG-3, and CTLA-4 can include antisense nucleic acids RNAs that interfere with the expression of TIM-3, PD-1, PDL-1, LAG-3, and CTLA-4; and small interfering RNAs that interfere with the expression of TIM-3, PD-1, PDL-1, LAG-3, and CTLA-4.

Candidate antagonists of TIM-3, PD-1, PDL-1, LAG-3, and CTLA-4 can be screened for function by a variety of techniques known in the art and/or disclosed within the instant application, such as ability to interfere with TIM-3, PD-1, PDL-1, LAG-3, and CTLA-4 function in an in vitro or mouse model.

### Antibodies

In one embodiment, the antagonist of TIM-3, PD-1, PDL-1, LAG-3, and CTLA-4 is an antibody. Antibodies can be synthetic, monoclonal, or polyclonal and can be made by techniques well known in the art. Such antibodies specifically bind to TIM-3, PD-1, LAG-3, PDL-1, and CTLA-4 via the antigen-binding sites of the antibody (as opposed to non-specific binding). TIM-3, PD-1, PDL-1, LAG-3, and CTLA-4 polypeptides, fragments, variants, fusion proteins, etc., can be employed as immunogens in producing antibodies immunoreactive therewith. More specifically, the polypeptides, fragment, variants, fusion proteins, etc. contain antigenic determinants or epitopes that elicit the formation of antibodies.

These antigenic determinants or epitopes can be either linear or conformational (discontinuous). Linear epitopes are composed of a single section of amino acids of the polypeptide, while conformational or discontinuous epitopes are composed of amino acids sections from different regions of the polypeptide chain that are brought into close proximity upon protein folding (C. A. Janeway, Jr. and P. Travers, Immuno Biology 3:9 (Garland Publishing Inc., 2nd ed. 1996)). Because folded proteins have complex surfaces, the number of epitopes available is quite numerous; however, due to the conformation of the protein and steric hinderances, the number of antibodies that actually bind to the epitopes is less than the number of available epitopes (C. A. Janeway, Jr. and P. Travers, Immuno Biology 2:14 (Garland Publishing Inc., 2nd ed. 1996)). Epitopes can be identified by any of the methods known in the art.

Antibodies, including scFV fragments, which bind specifically to TIM-3, PD-1, PDL-1, LAG-3, and CTLA-4 and block its function ("antagonist antibodies") are encompassed by the invention. Such antibodies can be generated by conventional means.

In one embodiment, the invention encompasses monoclonal antibodies against TIM-3, PD-1, PDL-1, LAG-3, and CTLA-4 that block each immune checkpoint molecule's function ("antibodies"). Exemplary blocking monoclonal antibodies against PD-1 are described in WO 2011/041613.

Antibodies are capable of binding to their targets with both high avidity and specificity. They are relatively large molecules (∼150kDa), which can sterically inhibit interactions between two proteins (e.g. PD-1 and its target ligand) when the antibody binding site falls within proximity of the protein-protein interaction site. The invention further encompasses antibodies that bind to epitopes within close proximity to a TIM-3, PD-1, PDL-1, LAG-3, or CTLA-4 ligand binding site.

In various embodiments, the invention encompasses antibodies that interfere with intermolecular interactions (e.g. protein-protein interactions), as well as antibodies that perturb intramolecular interactions (e.g. conformational changes within a molecule). Antibodies can be screened for the ability to block the biological activity of TIM-3, PD-1, PDL-1, LAG-3, or CTLA-4, or the binding of TIM-3, PD-1, PDL-1, LAG-3, or CTLA-4 to a ligand, and/or for other properties.

Both polyclonal and monoclonal antibodies can be prepared by conventional techniques.

TIM-3, PD-1, PDL-1, LAG-3, and CTLA-4 and peptides based on the amino acid sequence of TIM-3, PD-1, PDL-1, LAG-3, and CTLA-4 can be utilized to prepare antibodies that specifically bind to TIM-3, PD-1, PDL-1, LAG-3, and CTLA-4. The term "antibodies" is meant to include polyclonal antibodies, monoclonal antibodies, fragments thereof, such as F(ab')2 and Fab fragments, single-chain variable fragments (scFvs), single-domain antibody fragments (VHHs or Nanobodies), bivalent antibody fragments (diabodies), as well as any recombinantly and synthetically produced binding partners.

Antibodies are defined to be specifically binding if they bind TIM-3, PD-1, PDL-1, LAG-3, or CTLA-4 with a Ka of greater than or equal to about 10⁷ M⁻¹. Affinities of binding partners or antibodies can be readily determined using conventional techniques, for example those described by Scatchard et al., Ann. N.Y. Acad. Sci., 51:660 (1949).

Polyclonal antibodies can be readily generated from a variety of sources, for example, horses, cows, goats, sheep, dogs, chickens, rabbits, mice, or rats, using procedures that are well known in the art. In general, purified TIM-3, PD-1, PDL-1, LAG-3, and CTLA-4 or a peptide based on the amino acid sequence of TIM-3, PD-1, PDL-1, LAG-3, or CTLA-4 that is appropriately conjugated is administered to the host animal typically through parenteral injection. The immunogenicity of TIM-3, PD-1, PDL-1, LAG-3, and CTLA-4 can be enhanced through the use of an adjuvant, for example, Freund's complete or incomplete adjuvant. Following booster immunizations, small samples of serum are collected and tested for reactivity to TIM-3, PD-1, PDL-1, LAG-3, and CTLA-4 polypeptide. Examples of various assays useful for such determination include those described in Antibodies: A Laboratory Manual, Harlow and Lane (eds.), Cold Spring Harbor Laboratory Press, 1988; as well as procedures, such as countercurrent immuno-electrophoresis (CIEP), radioimmunoassay, radio-immunoprecipitation, enzyme-linked immunosorbent assays (ELISA), dot blot assays, and sandwich assays. See U.S. Pat. Nos. 4,376,110 and 4,486,530.

Monoclonal antibodies can be readily prepared using well known procedures. See, for example, the procedures described in U.S. Pat. Nos. RE 32,011, 4,902,614, 4,543,439, and 4,411,993; Monoclonal Antibodies, Hybridomas: A New Dimension in Biological Analyses, Plenum Press, Kennett, McKeam, and Bechtol (eds.), 1980.

For example, the host animals, such as mice, can be injected intraperitoneally at least once and preferably at least twice at about 3 week intervals with isolated and purified TIM-3, PD-1, PDL-1, LAG-3, and CTLA-4 or conjugated TIM-3, PD-1, PDL-1, LAG-3, and CTLA-4, optionally in the presence of adjuvant. Mouse sera are then assayed by conventional dot blot technique or antibody capture (ABC) to determine which animal is best to fuse. Approximately two to three weeks later, the mice are given an intravenous boost of TIM-3, PD-1, PDL-1, LAG-3, and CTLA-4 or conjugated TIM-3, PD-1, PDL-1, LAG-3, and CTLA-4 peptide. Mice are later sacrificed and spleen cells fused with commercially available myeloma cells, such as Ag8.653 (ATCC), following established protocols. Briefly, the myeloma cells are washed several times in media and fused to mouse spleen cells at a ratio of about three spleen cells to one myeloma cell. The fusing agent can be any suitable agent used in the art, for example, polyethylene glycol (PEG). Fusion is plated out into plates containing media that allows for the selective growth of the fused cells. The fused cells can then be allowed to grow for approximately eight days. Supernatants from resultant hybridomas are collected and added to a plate that is first coated with goat anti-mouse Ig. Following washes, a label, such as a labeled TIM-3, PD-1, PDL-1, LAG-3, and CTLA-4 polypeptide, is added to each well followed by incubation. Positive wells can be subsequently detected. Positive clones can be grown in bulk culture and supernatants are subsequently purified over a Protein A column (Pharmacia).

The monoclonal antibodies of the invention can be produced using alternative techniques, such as those described by Alting-Mees et al., "Monoclonal Antibody Expression Libraries: A Rapid Alternative to Hybridomas", Strategies in Molecular Biology 3:1-9 (1990), which is incorporated herein by reference. Similarly, binding partners can be constructed using recombinant DNA techniques to incorporate the variable regions of a gene that encodes a specific binding antibody. Such a technique is described in Larrick et al., Biotechnology, 7:394 (1989).

Antigen-binding fragments of such antibodies, which can be produced by conventional techniques, are also encompassed by the present invention. Examples of such fragments include, but are not limited to, Fab and F(ab')2 fragments. Antibody fragments and derivatives produced by genetic engineering techniques are also provided.

The monoclonal antibodies of the present invention include chimeric antibodies, e.g., humanized versions of murine monoclonal antibodies. Such humanized antibodies can be prepared by known techniques, and offer the advantage of reduced immunogenicity when the antibodies are administered to humans. In one embodiment, a humanized monoclonal antibody comprises the variable region of a murine antibody (or just the antigen binding site thereof) and a constant region derived from a human antibody. Alternatively, a humanized antibody fragment can comprise the antigen binding site of a murine monoclonal antibody and a variable region fragment (lacking the antigen-binding site) derived from a human antibody. Procedures for the production of chimeric and further engineered monoclonal antibodies include those described in Riechmann et al. (Nature 332:323, 1988), Liu et al. (PNAS 84:3439, 1987), Larrick et al. (Bio/Technology 7:934, 1989), and Winter and Harris (TIPS 14:139, May, 1993). Procedures to generate antibodies transgenically can be found in GB 2,272,440, U.S. Pat. Nos. 5,569,825 and 5,545,806.

Antibodies produced by genetic engineering methods, such as chimeric and humanized monoclonal antibodies, comprising both human and non-human portions, which can be made using standard recombinant DNA techniques, can be used. Such chimeric and humanized monoclonal antibodies can be produced by genetic engineering using standard DNA techniques known in the art, for example using methods described in Robinson et al. International Publication No. WO 87/02671; Akira, et al. European Patent Application 0184187; Taniguchi, M., European Patent Application 0171496; Morrison et al. European Patent Application 0173494; Neuberger et al. PCT International Publication No. WO 86/01533; Cabilly et al. U.S. Pat. No. 4,816,567; Cabilly et al. European Patent Application 0125023; Better et al., Science 240:1041 1043, 1988; Liu et al., PNAS 84:3439 3443, 1987; Liu et al., J. Immunol. 139:3521 3526, 1987; Sun et al. PNAS 84:214 218, 1987; Nishimura et al., Canc. Res. 47:999 1005, 1987; Wood et al., Nature 314:446 449, 1985; and Shaw et al., J. Natl. Cancer Inst. 80:1553 1559, 1988); Morrison, S. L., Science 229:1202 1207, 1985; Oi et al., BioTechniques 4:214, 1986; Winter U.S. Pat. No. 5,225,539; Jones et al., Nature 321:552 525, 1986; Verhoeyan et al., Science 239:1534, 1988; and Beidler et al., J. Immunol. 141:4053 4060, 1988.

In connection with synthetic and semi-synthetic antibodies, such terms are intended to cover but are not limited to antibody fragments, isotype switched antibodies, humanized antibodies (e.g., mouse-human, human-mouse), hybrids, antibodies having plural specificities, and fully synthetic antibody-like molecules.

For therapeutic applications, "human" monoclonal antibodies having human constant and variable regions are often preferred so as to minimize the immune response of a patient against the antibody. Such antibodies can be generated by immunizing transgenic animals which contain human immunoglobulin genes. See Jakobovits et al. Ann NY Acad Sci 764:525-535 (1995).

Human monoclonal antibodies against TIM-3, PD-1, PDL-1, LAG-3, and CTLA-4 polypeptides can also be prepared by constructing a combinatorial immunoglobulin library, such as a Fab phage display library or a scFv phage display library, using immunoglobulin light chain and heavy chain cDNAs prepared from mRNA derived from lymphocytes of a subject. See, e.g., McCafferty et al. PCT publication WO 92/01047; Marks et al. (1991) J. Mol. Biol. 222:581 597; and Griffths et al. (1993) EMBO J 12:725 734. In addition, a combinatorial library of antibody variable regions can be generated by mutating a known human antibody. For example, a variable region of a human antibody known to bind TIM-3, PD-1, PDL-1, LAG-3, and CTLA-4 can be mutated, by for example using randomly altered mutagenized oligonucleotides, to generate a library of mutated variable regions which can then be screened to bind to TIM-3, PD-1, PDL-1, LAG-3, and CTLA-4. Methods of inducing random mutagenesis within the CDR regions of immunoglobin heavy and/or light chains, methods of crossing randomized heavy and light chains to form pairings and screening methods can be found in, for example, Barbas et al. PCT publication WO 96/07754; Barbas et al. (1992) Proc. Nat'l Acad. Sci. USA 89:4457 4461.

An immunoglobulin library can be expressed by a population of display packages, preferably derived from filamentous phage, to form an antibody display library. Examples of methods and reagents particularly amenable for use in generating antibody display library can be found in, for example, Ladner et al. U.S. Pat. No. 5,223,409; Kang et al. PCT publication WO 92/18619; Dower et al. PCT publication WO 91/17271; Winter et al. PCT publication WO 92/20791; Markland et al. PCT publication WO 92/15679; Breitling et al. PCT publication WO 93/01288; McCafferty et al. PCT publication WO 92/01047; Garrard et al. PCT publication WO 92/09690; Ladner et al. PCT publication WO 90/02809; Fuchs et al. (1991) Bio/Technology 9:1370 1372; Hay et al. (1992) Hum Antibod Hybridomas 3:81 85; Huse et al. (1989) Science 246:1275 1281; Griffths et al. (1993) supra; Hawkins et al. (1992) J Mol Biol 226:889 896; Clackson et al. (1991) Nature 352:624 628; Gram et al. (1992) PNAS 89:3576 3580; Garrad et al. (1991) Bio/Technology 9:1373 1377; Hoogenboom et al. (1991) Nuc Acid Res 19:4133 4137; and Barbas et al. (1991) PNAS 88:7978 7982. Once displayed on the surface of a display package (e.g., filamentous phage), the antibody library is screened to identify and isolate packages that express an antibody that binds a TIM-3, PD-1, PDL-1, LAG-3, or CTLA-4 polypeptide. In a preferred embodiment, the primary screening of the library involves panning with an immobilized TIM-3, PD-1, PDL-1, LAG-3, and CTLA-4 polypeptide and display packages expressing antibodies that bind immobilized TIM-3, PD-1, PDL-1, LAG-3, and CTLA-4 polypeptide are selected.

In addition to the TIM-3, PD-1, PDL-1, LAG-3, and CTLA-4 antagonists already described herein, it is contemplated that the antagonists and agonists described herein can include those known in the art. For example, Ipilimumab® and tremelimumab, are known CTLA-4 antibodies. Additionally, lambrolizumab, AMP-224, Nivolumab, and MK-3475 are known PD-1 antibodies. Some exemplary known antibodies for PDL-1 that interfere with PD-1 function include: MPDL3280A (Roche), MED14736 (AZN), MSB0010718C (Merck).

### Combination Therapy with a Poxvirus Expressing a Tumor Antigen and an Immune Checkpoint Antagonist or Agonist

In at least one aspect, the invention encompasses a combination of a recombinant poxvirus encoding a tumor antigen with one or more immune checkpoint antagonists or agonists, for use in methods of treatment.

In at least one aspect, the invention encompasses a combination of a recombinant poxvirus encoding a TAA and one or more TIM-3 antibodies or antagonists, for use in cancer treatment.

In another aspect, the invention encompasses a combination of (a) a recombinant poxvirus encoding a TAA; (b) one or more TIM-3 antibodies or antagonists; and (c) and one or more other immune checkpoint molecule antibodies, agonists, or antagonists, for use in methods of treatment. In a preferred embodiment the other immune checkpoint molecule antibodies, agonists, or antagonists are selected from antibodies or antagonists of PD-1, PDL-1, LAG-3, or CTLA-4, or combinations thereof.

In one embodiment, patients with a cancer mediated by cells over-expressing the tumor-associated antigen HER-2 (e.g., breast cancer) can be treated by the combination of a poxvirus, for example an orthopoxvirus (e.g., vaccinia virus, Wyeth, ACAM 1000, ACAM 2000, MVA, or MVA-BN) or an avipoxvirus (e.g.,fowlpoxvirus, POXVAC-TC), encoding a HER-2 antigen with one or more antibodies, agonists, or antagonists according to the invention. In a preferred embodiment, the MVA is MVA-BN. In a particularly preferred embodiment, the MVA encodes a polypeptide comprising SEQ ID NO:2.

In one embodiment, patients with a prostate cancer can be treated by the combination of an orthopoxvirus, for example a vaccinia virus (e.g., vaccinia virus, Wyeth, ACAM 1000, ACAM 2000, MVA, or MVA-BN) and an avipoxvirus (e.g., fowlpoxvirus. POXVAC-TC), encoding a PSA and/or PAP antigen, with one or more antibodies, agonists, or antagonists according to the invention. In a particularly preferred embodiment, the Vaccinia virus is part of PROSTVAC®.

In one embodiment, patients with a cancer mediated by cells over-expressing the TAA CEA and/or MUC-1 (e.g., breast, colorectal, lung, and ovarian cancer) can be treated by the combination of an orthopoxvirus, for example a vaccinia virus (e.g., vaccinia virus, Wyeth, ACAM 1000, ACAM 2000, MVA, or MVA-BN) or an avipoxvirus (e.g. fowlpoxvirus, POXVAC-TC), encoding a CEA and/or MUC-1 antigen, with one or more antibodies, agonists, or antagonists according to the invention..

The recombinant poxvirus can be administered either systemically or locally, i.e., by parenteral, subcutaneous, intravenous, intramuscular, intranasal, intradermal, scarification, or any other path of administration known to a skilled practitioner. Preferably, the administration is via scarification. In one embodiment, 10⁵⁻10¹⁰ TCID₅₀ of the recombinant poxvirus are administered to the patient. Preferably, 10⁷⁻10¹⁰ TCID₅₀ of the recombinant poxvirus are administered to the patient. More preferably, 10⁸⁻10¹⁰ TCID₅₀ of the recombinant poxvirus are administered to the patient. Most preferably, 10⁸⁻10⁹ TCID₅₀ of the recombinant poxvirus are administered to the patient.

The cancer preferably includes, but is not limited to, a breast cancer, lung cancer, head and neck cancer, thyroid, melanoma, gastric cancer, bladder cancer, kidney cancer, liver cancer, melanoma, pancreatic cancer, prostate cancer, ovarian cancer, or colorectal cancer.

. In a preferred embodiment, the cancer is a breast cancer, prostate cancer, or colorectal cancer.

The cancer patient can be any mammal, including a mouse or rat. Preferably, the cancer patient is a primate, most preferably, a human.

In one embodiment, one or more antibodies, agonist or antagonist, according to the invention and the poxvirus encoding a polypeptide comprising a TAA are administered at the same time. The combination treatment is superior to either treatment alone.

In preferred embodiments, the recombinant poxvirus is for administration within 1, 2, 3, 4, 5, 6, or 7, days of agonist and/or antagonist administration. The recombinant poxvirus can be administered before or after the agonist and/or antagonist.

The dosage of agonist or antagonist administered to a patient is typically 0.1 mg/kg to 100 mg/kg of the patient's body weight. Preferably, the dosage administered to a patient is between 0.1 mg/kg and 20 mg/kg of the patient's body weight, more preferably 1 mg/kg to 10 mg/kg of the patient's body weight, most preferably 3 mg/kg to 10 mg/kg of the patient's body weight. Generally, human and humanized antibodies have a longer half-life within the human body than antibodies from other species due to the immune response to the foreign polypeptides. Thus, lower dosages of human antibodies and less frequent administration is often possible.

The quantities of active ingredient necessary for effective therapy will depend on many different factors, including means of administration, target site, physiological state of the patient, and other medicaments administered. Thus, treatment dosages should be titrated to optimize safety and efficacy. Typically, dosages used in vitro can provide useful guidance in the amounts useful for in situ administration of the active ingredients. Animal testing of effective doses for treatment of particular disorders will provide further predictive indication of human dosage. Various considerations are described, for example, in Goodman and Gilman's the Pharmacological Basis of Therapeutics, 7th Edition (1985), MacMillan Publishing Company, New York, and Remington's Pharmaceutical Sciences 18th Edition, (1990) Mack Publishing Co, Easton Pa. Methods for administration are discussed therein, including oral, intravenous, intraperitoneal, intramuscular, transdermal, nasal, iontophoretic administration, and the like.

The compositions of the invention can be administered in a variety of unit dosage forms depending on the method of administration. For example, unit dosage forms suitable for oral administration include solid dosage forms such as powder, tablets, pills, capsules, and dragees, and liquid dosage forms, such as elixirs, syrups, and suspensions. The active ingredients can also be administered parenterally in sterile liquid dosage forms. Gelatin capsules contain the active ingredient and as inactive ingredients powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that can be added to provide desirable color, taste, stability, buffering capacity, dispersion or other known desirable features are red iron oxide, silica gel, sodium lauryl sulfate, titanium dioxide, edible white ink and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain coloring and flavoring to increase patient acceptance.

The concentration of the compositions of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

For solid compositions, conventional nontoxic solid carriers can be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more compositions of the invention of the invention, and more preferably at a concentration of 25%-75%.

For aerosol administration, the compositions of the invention are preferably supplied in finely divided form along with a surfactant and propellant. Preferred percentages of compositions of the invention are 0.01%-20% by weight, preferably 1-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides can be employed. The surfactant can constitute 0.1%-20% by weight of the composition, preferably 0.25-5%. The balance of the composition is ordinarily propellant. A carrier can also be included, as desired, as with, e.g., lecithin for intranasal delivery.

The constructs of the invention can additionally be delivered in a depot-type system, an encapsulated form, or an implant by techniques well-known in the art. Similarly, the constructs can be delivered via a pump to a tissue of interest.

Any of the foregoing formulations can be appropriate in treatments and therapies in accordance with the present invention, provided that the active agent in the formulation is not inactivated by the formulation and the formulation is physiologically compatible.

In certain embodiments, the recombinant poxviruses of the present invention can be embodied in one or more pharmaceutical compositions. In addition to a recombinant poxvirus encoding a TAA and one or more immune checkpoint antagonists or agonists, pharmaceutical compositions may comprise one or more pharmaceutically acceptable and/or approved carriers, additives, antibiotics, preservatives, adjuvants, diluents and/or stabilizers. Such additives include, for example, but not limited to, water, saline, glycerol, ethanol, wetting or emulsifying agents, and pH buffering substances. Exemplary carriers are typically large, slowly metabolized molecules such as proteins, polysaccharides, polylactic acids, polyglycolic acids, polymeric amino acids, amino acid copolymers, lipid aggregates, or the like.

### Combination therapy using homologous/heterologous prime-boost regimens

It is possible to induce an immune response with a single administration of the recombinant poxvirus as defined above. The poxvirus according to the present invention may also be used as part of a homologous prime-boost regimen. In the homologous prime-boost, a first priming vaccination is given followed by one or more subsequent boosting vaccinations. The boosting vaccinations are configured to boost the immune response generated in the first vaccination by administration of the same or a related recombinant poxvirus that was used in the first vaccination.

The recombinant poxvirus according to the present invention may also be used in heterologous prime-boost regimens in which one or more of the initial prime vaccinations are done with a poxvirus as defined herein and in which one or more subsequent boosting vaccinations is done with a different vaccine, such as but not limited to, another virus vaccine, a protein or a nucleic acid vaccine.

In one exemplary embodiment, a homologous prime-boost regimen may be employed wherein a poxvirus such as an MVA-BN expressing one or more Tumor Associated Antigens (TAAs), such as, but not limited to HER2, is administered in a first dosage in combination with one or more immune checkpoint antagonists or agonists. One or more Subsequent administrations of MVA-BN expressing one or more TAAs, such as, but not limited to HER2, in combination with one or more immune checkpoint antagonists or agonists can be given to boost the immune response provide in the first administration. Preferably, the one or more TAAs in the second and subsequent MVA-BNs are the same or similar TAAs to those of the first administration.

In another exemplary embodiment, a heterologous prime-boost may be employed wherein a poxvirus such as vaccinia expressing one or more TAAs is administered in a first dose in combination with one or more immune checkpoint antagonist or agonists. This first dose is followed by one or more administrations of different poxvirus such as fowlpox expressing one or more TAAs. Preferably, the one or more TAAs in the fowlpox virus are the same or similar TAAs to those included in the vaccinia of the first administration. Further description of exemplary heterologous prime-boost regimens can be found in US Patents 6,165,460; 7,598,225; and 7,247,615.

In one preferred embodiment, the one or more TAAs in the heterologous prime-boost regimen include prostate specific antigen (PSA) and/or prostatic acid phosphatase (PAP) antigen. In a more preferred embodiment, the PSA antigen can include those PSA antigens found in US Patents 7,247,615 and 7,598,225. In one non-limiting example, the heterologous prime-boost including PSA is PROSTVAC®.

In yet another preferred embodiment, the one or more TAAs in the heterologous prime-boost regimen include A mucin 1, cell surface associated (MUC1) antigen and a carcinoembryonic antigen (CEA). In a more preferred embodiment, the MUC1 and the CEA antigens can include those found in US Patents 7,118,738; 7,723,096; and PCT application No. PCT/US2013/020058. In one non-limiting example, the heterologous prime-boost regimen including a MUC-1 antigen and CEA is CV301.

In yet another exemplary embodiment, a heterologous prime-boost may be employed wherein a poxvirus, such as MVA or MVA-BN, expressing one or more TAAs is administered in a first dose in combination with one or more immune checkpoint antagonists or agonists. This first dose is followed by one or more administrations of different poxvirus, such as fowlpox, expressing one or more TAAs. Preferably, the one or more TAAs in the fowlpox virus are the same or similar TAAs to those included in the MVA or MVA-BN virus of the first administration.

In certain embodiments, the one or more boosting vaccinations are administered at intervals comprising days, weeks or months after administration of the initial priming vaccination. In certain embodiments, the one or more boosting vaccinations are administered at intervals of the same day, or 1, 2, 3, 4, 5, 6, 7 or more days after administration of the initial priming vaccination. In certain embodiments, the one or more boosting vaccinations are administered at intervals of 1, 2, 3, 4, 5, 6, 7, 8 or more weeks after administration of the initial priming vaccination. In certain embodiments, the one or more boosting vaccinations are administered at intervals of 1, 2, 3, 4, 5, 6, 7, 8, 9, 10, 11, 12 or more months after administration of the initial priming vaccination. In certain embodiments, the one or more boosting vaccinations are administered at any combination of intervals after administration of the initial the priming vaccination)(e.g., 1, 2, 3, 4, 5, 6, 7 or more days, 1, 2, 3, 4, 5, 6, 7, 8 or more weeks, or 1, 2, 3, 4, 5, 6, 7, 8, 9, 10, 11, 12 or more months).

In one embodiment, the one or more subsequent boosting vaccinations of a heterologous prime-boost regimen are selected from poxviruses of a different genus than the initial prime vaccinations. In one non-limiting example, when the first or initial pox virus vaccine includes vaccinia, the second and subsequent poxvirus vaccines are selected from the poxviruses from a different genus such as suipox, avipox, capripox or an orthopox immunogenically different from vaccinia.

### Administering an Immune Checkpoint Antagonist/Agonist after Boost Dosages in Heterologous Prime-Boost Increases Efficacy of Cancer Treatments

PROSTVAC® comprises a heterologous prime-boost regimen that includes a single prime administration with PROSTVAC-V (Vaccinia virus expressing PSA and TRICOMTM) followed by one or more consecutive boosting doses of PROSTVAC-F (Fowlpoxvirus expressing PSA and TRICOMTM); also described in J Clin Oncol 2010, 28:1099-1105.

As shown and described in Figures 10-12 and Examples 13-15, a heterologous PROSTVAC® dosing regimen greatly enhances the magnitude and quality of the PSA-specific T cell response as compared to homologous dosing with the same vector. Additionally, the figures and examples demonstrate that priming with PROSTVAC-V and boosting with PROSTVAC-F provides the added benefit of focusing the highly functional CD8 CTL immune response towards PSA, the target tumor antigen, and away from the vaccinia vector.

In at least in one aspect, when one or more dosages of a recombinant poxvirus are administered to a cancer patient, greater therapeutic efficacy in the cancer treatment is achieved by administering one or more immune checkpoint antagonists or agonists in combination with a second or subsequent recombinant poxvirus dosage or administration.

In a more particular aspect, when one or more dosages of a recombinant poxvirus of the present invention are administered to a cancer patient as part of a heterologous prime-boost regimen greater therapeutic efficacy in the cancer treatment is achieved by administering at least one checkpoint antagonist or agonist in combination with the second or subsequent boost dosages of recombinant poxvirus encoding at least one TAA. This greater therapeutic efficacy is realized, at least in part, because during and after the second or subsequent boost dosages of a heterologous prime-boost regimen a patient's immune T-cell response is more focused on the tumor antigen as compared to the recombinant poxvirus. Accordingly, at least in one aspect, an administration of an immune checkpoint antagonist or agonist during the boosting dosages functions to enhance a patient's immune response to the tumor antigen, and thereby increase a patient's immune response more specifically to the tumor.

In at least another aspect, as part of a cancer treatment involving a heterologous prime-boost regimen, administering at least one immune checkpoint antagonist or agonist in combination with the second or subsequent boost dosages of recombinant poxvirus maximizes therapeutic benefits of the immune checkpoint antagonist or agonist while minimizing adverse side effects that have been seen in the immune checkpoint treatments.

In view of the teachings of the present disclosure, in additional embodiments, the present invention includes a combination for use in treating a human cancer patient, the combination comprising: (a) a first recombinant poxvirus, the poxvirus comprising at least one tumor-associated antigen (TAA); and (b) a second recombinant poxvirus, the poxvirus comprising at least one tumor-associated antigen (TAA); wherein the second recombinant poxvirus is administered in combination with at least one immune checkpoint antagonist or agonist. In an additional embodiment, the second recombinant poxvirus is different than the first recombinant poxvirus. In other embodiments, the second recombinant poxvirus is from a different genus than the first recombinant poxvirus.

In another embodiment, the first and second recombinant poxviruses are different or are of a different genus and are administered as a heterologous prime-boost regimen, the heterologous prime-boost regimen comprising: a) administering the first recombinant poxvirus as a first prime dose; and b) administering the second recombinant poxvirus as one or more boost doses in combination with at least one immune checkpoint antagonist or agonist. In a preferred embodiment, the heterologous prime boost regimen is selected from PROSTVAC®, CV301 or MVA-BN-CV301.

In yet another embodiment, it is contemplated that the first recombinant poxvirus or the recombinant poxvirus of the initial or prime dose does not include an immune checkpoint antagonist or agonist.

It is additionally contemplated that the first and second recombinant poxviruses can be any poxvirus, such as but not limited to, those described in the present disclosure. It is further contemplated that the at least one tumor-associated antigen (TAA) can be any TAA, such as but not limited to, those TAAs described in the present disclosure.

In one or more embodiments, at least one immune checkpoint antagonist or agonist is administered on the same day or within 1, 2, 3, 4, 5, 6, or 7, days of the second or subsequent dosages of a recombinant poxvirus encoding at least one TAA. In a preferred embodiment, at least one immune checkpoint antagonist or agonist is administered as part of a heterologous prime-boost regimen and is administered on the same day or within 1, 2, 3, 4, 5, 6, or 7, days of the second or subsequent boost dosages of a recombinant poxvirus encoding at least one TAA.

In one or more embodiments, at least one immune checkpoint antagonist or agonist is administered after the second or subsequent dosages of a recombinant poxvirus encoding at least one TAA is administered. In a preferred embodiment, at least one immune checkpoint antagonist or agonist is administered as part of a heterologous prime-boost regimen, and is administered after the second or subsequent boost dosages of a recombinant poxvirus encoding at least one TAA. It is contemplated that, after the second or subsequent boost dosages of a recombinant poxvirus, the time intervals at which at least one immune checkpoint antagonist or agonist is administered can include those time intervals described in the present disclosure.

It is additionally contemplated that when administered in combination with a second or one more subsequent boost dosages of a recombinant poxvirus encoding at least one TAA, at least one immune checkpoint antagonist or agonist can be administered at a dosage or concentration as provided in the present disclosure.

### Additional Embodiments for Combinations or Medicaments

In additional embodiments, the present disclosure encompasses a combination or medicament for use in treating a human cancer patient. The combination or medicament comprises a recombinant poxvirus vector, the poxvirus vector comprising a) at least one tumor associated antigen (TAA); and b) a TIM-3 antagonist. The TIM-3 antagonist can include an anti-TIM-3 antibody, antisense RNA, or siRNA.

In still an additional embodiment, the present disclosure can include a combination or medicament for use in treating a human cancer patient, the combination or medicament comprising: (a) a therapeutically effective amount of a recombinant poxvirus, the poxvirus vector comprising at least one tumor associated antigen (TAA); (b) a therapeutically effective amount of at least one TIM-3 antagonist; and (c) a therapeutically effective amount of at least one of a PD-1 antagonist, a LAG-3 antagonist, or a CTLA-4 antagonist. It is contemplated that the various immune checkpoint antagonists or agonists can be embodied in one or more antibodies, antisense RNAs, or siRNAs.

In still another embodiment, the present disclosure can include a combination or medicament for use in increasing overall survival rate in a human cancer patient, the combination or medicament comprising: (a) a recombinant poxvirus vector, the poxvirus vector comprising at least one tumor associated antigen (TAA); and b) a TIM-3 antagonist. The TIM-3 antagonist can include an anti-TIM-3 antibody.

In still another embodiment, the recombinant poxvirus encoding a TAA in the combination or medicaments described herein can be PROSTVAC®. In yet another embodiment, the recombinant poxvirus encoding a TAA in the combination or medicaments described herein can be CV301.

In still an additional embodiment, the present disclosure can include use of: (a) a recombinant poxvirus, the poxvirus comprising at least one tumor associated antigen (TAA); and (b) a TIM-3 antagonist. The TIM-3 antagonist can include an anti-TIM-3 antagonist antibody. In an additional embodiment, the use of the disclosed pharmaceutical composition or medicament can be for the treatment of a human cancer patient.

In still an additional embodiment, the present disclosure can include use of: (a) a recombinant poxvirus, the poxvirus comprising at least one tumor associated antigen (TAA); and (b) a TIM-3 antagonist; and (c) at least one of a PD-1 antagonist, a LAG-3 antagonist, or a CTLA-4 antagonist. It is contemplated that the various immune checkpoint antagonists or agonists can be embodied in one or more antibodies. In an additional embodiment, the use of the disclosed pharmaceutical composition or medicament can be for the treatment of a human cancer patient.

### EXAMPLES

### Example 1

### Construction of MVA-BN-HER2

Simultaneous infection and transfection of cultures allowed homologous recombination to occur between the viral genome and the recombination plasmid. Insert-carrying virus was isolated, characterized, and virus stocks were prepared.

Plasmid pBN146 contains sequences which are also present in MVA-BN (the 14L and 15L open reading frames). The HER2 sequence was inserted between the MVA-BN sequences to allow for recombination into the MVA-BN viral genome. Thus, a plasmid was constructed that contained the HER2 sequence downstream of a poxvirus promoter, specifically the cowpox virus A-type inclusion body gene promoter. The plasmid also contained a selection cassette comprising a synthetic vaccinia virus promoter (Ps), a drug resistance gene (guanine-xanthine phosphoribosyltransferase; Ecogpt), an internal ribosomal entry site (IRES), and the enhanced green fluorescent protein (EGFP). Both selection genes (gpt and EGFP) were encoded by a single bicistronic transcript.

The HER-2 sequence was modified by addition of nucleotides sequences encoding tetanus toxin epitopes of p2 and p30 to increase the immune response against it. After mHER2 was inserted into the MVA-BN genome, the virus "insert region" had the following structure:

ATI promoter - HER2 sequence - Ps promoter - gpt - IRES - EGFP. The insert region was flanked by MVA-BN I4L intergenic region sequences (F1 and F2) in the bacterial recombination plasmid pBN146. The nucleotide sequence of the construct is shown below.

AGTATGCATTTTTACGGATGGAGTCTCGGTCTAAAAACGGGAATGTACTATCTACGTACG
AAACCCGCATCCGCTCCCATTCAATTCACATTGGACAAGGATAAAATAAAACCACTGGTG
GTTTGCGATTCCGAAATCTGTACATCATGCAGTGGTTAAACAAATCTAGAACTAGTTTAA
TTAAGGAGCTGTTTTGAATAAAATTTTTTTATAATAAATCTAGAACTAGTGGATCCCCCG
GGCTGCAGGAATTCGATCTAGCCGCCACCATGGAGCTGGCGGCCTTGTGCCGCTGGGGGC
TCCTCCTCGCCCTCTTGCCCCCCGGAGCCGCGAGCACCCAAGTGTGCACCGGCACAGACA
TGAAGCTGCGGCTCCCTGCCAGTCCCGAGACCCACCTGGACATGCTCCGCCACCTCTACC
AGGGCTGCCAGGTGGTGCAGGGAAACCTGGAACTCACCTACCTGCCCACCAATGCCAGCT
TAAGTTTCCTGCAGGATATCCAGGAGGTGCAGGGCTACGTGCTCATCGCTCACAACCAAG
TGAGGCAGGTCCCACTGCAGAGGCTGCGGATTGTGCGAGGCACCCAGCTCTTTGAGGACA
ACTATGCCCTGGCCGTGCTAGACAATGGAGACCCGCTGAACAATACCACCCCTGTCACAG
GGGCCTCCCCAGGAGGCCTGCGGGAGCTGCAGCTTCGAAGCCTCACAGAGATCTTGAAAG
GAGGGGTCTTGATCCAGCGGAACCCCCAGCTCTGCTACCAGGACACGATTTTGTGGAAGG
ACATCTTCCACAAGAACAACCAGCTGGCTCTCACACTGATAGACACCAACCGCTCTCGGG
CCTGCCACCCCTGTTCTCCGATGTGTAAGGGCTCCCGCTGCTGGGGAGAGAGTTCTGAGG
ATTGTCAGAGCCTGACGCGCACTGTCTGTGCCGGTGGCTGTGCCCGCTGCAAGGGGCCAC
TGCCCACTGACTGCTGCCATGAGCAGTGTGCTGCCGGCTGCACGGGCCCCAAGCACTCTG
ACTGCCTGGCCTGCCTCCACTTCAACCACAGTGGCATCTGTGAGCTGCACTGCCCAGCCC
TGGTCCAGTACATCAAAGCTAACTCCAAATTCATCGGTATCACCGAGCTGCGGTATACAT
TCGGCGCCAGCTGTGTGACTGCCTGTCCCTACAACTACCTTTCTACGGACGTGGGATCCT
GCACCCTCGTCTGCCCCCTGCACAACCAAGAGGTGACAGCAGAGGATGGAACACAGCGGT
GTGAGAAGTGCAGCAAGCCCTGTGCCCGAGTGTGCTATGGTCTGGGCATGGAGCACTTGC
GAGAGGTGAGGGCAGTTACCAGTGCCAATATCCAGGAGTTTGCTGGCTGCAAGAAGATCT
TTGGGAGCCTGGCATTTCTGCCGGAGAGCTTTGATGGGGACCCAGCCTCCAACACTGCCC
CGCTCCAGCCAGAGCAGCTCCAAGTGTTTGAGACTCTGGAAGAGATCACAGGTTACCTAT
ACATCTCAGCATGGCCGGACAGCCTGCCTGACCTCAGCGTCTTCCAGAACCTGCAAGTAA
TCCGGGGACGAATTCTGCACAATGGCGCCTACTCGCTGACCCTGCAAGGGCTGGGCATCA
GCTGGCTGGGGCTGCGCTCACTGAGGGAACTGGGCAGTGGACTGGCCCTCATCCACCATA
ACACCCACCTCTGCTTCGTGCACACGGTGCCCTGGGACCAGCTCTTTCGGAACCCGCACC
AAGCTCTGCTCCACACTGCCAACCGGCCAGAGGACGAGTGTGTGGGCGAGGGCCTGGCCT
GCCACCAGCTGTGCGCCCGAGGGCACTGCTGGGGTCCAGGGCCCACCCAGTGTGTCAACT
GCAGCCAGTTCCTTCGGGGCCAGGAGTGCGTGGAGGAATGCCGAGTACTGCAGGGGCTCC
CCAGGGAGTATGTGAATGCCAGGCACTGTTTGCCGTGCCACCCTGAGTGTCAGCCCCAGA
ATGGCTCAGTGACCTGTTTTGGACCGGAGGCTGACCAGTGTGTGGCCTGTGCCCACTATA
AGGACCCTCCCTTCTGCGTGGCCCGCTGCCCCAGCGGTGTGAAACCTGACCTCTCCTACA
TGCCCATCTGGAAGTTTCCAGATGAGGAGGGCGCATGCCAGCCTTGCCCCATCAACTGCA
CCCACTCCTGTGTGGACCTGGATGACAAGGGCTGCCCCGCCGAGCAGAGAGCCAGCCCTC
TGACGTCCTTCAACAACTTCACCGTGAGCTTCTGGCTGCGCGTGCCCAAGGTGAGCGCCA
GCCACCTGGAGATCGTCTCTGCGGTGGTTGGCATTCTGTAGAAGCTTGGTACCGAGCTCG
GATCCACTAGTCCAGTGTGGTGGAATTCTGCAGATATCCAGCACAGTGGCGGCCATCAAG
CTTATCGATACCGTCGACCTCGAGGGGGGGCCCGGTACCCAGTTAATTAAGGATCCCCCG

GGCTGCAGGAATTCCATTTTTATTCTCAAATGAGATAAAGTGAAAATATATATCATATAT
ACAAAGTA

(SEQ ID NO:1).

HER2 start and stop codons are indicated in bold. Flanking sequences are indicated in italics.

Translation of the encoded HER2 polypeptide is shown below:

MELAALCRWGLLLALLPPGAASTQVCTGTDMKLRLPASPETHLDMLRHLYQGCQVVQGNL
ELTYLPTNASLSFLQDIQEVQGYVLIAHNQVRQVPLQRLRIVRGTQLFEDNYALAVLDNG
DPLNNTTPVTGASPGGLRELQLRSLTEILKGGVLIQRNPQLCYQDTILWKDIFHKNNQLA
LTLIDTNRSRACHPCSPMCKGSRCWGESSEDCQSLTRTVCAGGCARCKGPLPTDCCHEQC
AAGCTGPKHSDCLACLHFNHSGICELHCPALVQYIKANSKFIGITELRYTFGASCVTACP
YNYLSTDVGSCTLVCPLHNQEVTAEDGTQRCEKCSKPCARVCYGLGMEHLREVRAVTSAN
IQEFAGCKKIFGSLAFLPESFDGDPASNTAPLQPEQLQVFETLEEITGYLYISAWPDSLP
DLSVFQNLQVIRGRILHNGAYSLTLQGLGISWLGLRSLRELGSGLALIHHNTHLCFVHTV
PWDQLFRNPHQALLHTANRPEDECVGEGLACHQLCARGHCWGPGPTQCVNCSQFLRGQEC
VEECRVLQGLPREYVNARHCLPCHPECQPQNGSVTCFGPEADQCVACAHYKDPPFCVARC
PSGVKPDLSYMPIWKFPDEEGACQPCPINCTHSCVDLDDKGCPAEQRASPLTSFNNFTVS
FWLRVPKVSASHLEIVSAVVGIL.

(SEQ ID NO:2).

The tetanus toxin epitopes of p2 and p30 sequences are indicated in bold.

CEF cultures were inoculated with MVA-BN and also transfected with pBN146 plasmid DNA. In turn, samples from these cell cultures were inoculated into CEF cultures in medium containing selection drugs, and EGFP-expressing viral clones were isolated by plaque purification. Virus stocks which grew in the presence of the selection drugs and expressed EGFP were designated MVA-BN-mHER2. Generation of MVA-BN-HER2 and preparation of the virus stock involved twelve (12) sequential passages, including five (5) plaque purifications.

MVA-BN-HER2 was passaged in CEF cell cultures in the absence of selection drugs. The absence of selection drugs allowed loss of the region encoding the selection genes, gpt and EGFP and the associated promoter (the selection cassette) from the inserted sequence. Recombination resulting in loss of the selection cassette is mediated by the F1 I4L region and a subsection of that region, the F1 repeat (F1 rpt), which flank the selection cassette in plasmid pBN146. These duplicated sequences were included to mediate recombination that results in loss of the selection cassette, leaving only the HER2 sequence inserted in the I4L intergenic region.

Plaque-purified virus lacking the selection cassette was prepared. Such preparation involved fifteen (15) passages including five (5) plaque purifications.

The presence of the HER2 sequence and absence of parental MVA-BN virus in MVA-BN-HER2 stocks was confirmed by PCR analysis, and nested PCR was used to verify the absence of the selection cassette (the gpt and EGFP genes).

Expression of the HER2 protein was demonstrated in cells inoculated with MVA-BN-HER2 *in vitro.*

### Example 2

### Tumor Implantation and treatment with MVA-BN-HER2 and antibodies

Female BALB/c mice (6-8 weeks old, ∼ 20 g) were purchased from Simonsen Laboratories, Gilroy, CA. In the solid tumor model, female BALB/c mice were implanted on day 1 with CT26-HER-2 cells (1.0×10^5, i.d. in the dorsal flank). Mice were treated on day 1 and 15 with MVA-BN-HER2 (1E7 Inf. U. in 100 µL TBS, by tail scarification [t.s.] or subcutaneously [s.c.] at the tail base). The following antibodies were purchased from Bio X Cell (West, Lebanon, NH): anti-Tim-3 (RMT3-23), anti-CTLA-4 (9D9), anti-PD-1 (RMP1-14), and anti-LAG-3 (C9B7W). All antibodies were injected i.p. at 200 µg per mouse in 100 µL PBS on the days 1 and 15 unless otherwise indicated. Tumors were measured twice weekly and the tumor volume calculated according to the formula: tumor volume (mm3) = (length × width2)/2.

Whole blood, tumor/lungs or spleens were pooled (4 mice/group) for flow cytometric analysis. Splenocytes were prepared by pressing the spleens between two frosted glass slides, and lysing the red blood cells with ACK lysis buffer (Life Technologies, Grand Island, NY). Lungs and associated tumors were diced to ∼1-2 mm³ pieces and further digested to single cell suspensions for 1 h at 37 °C in DMEM with 10% FBS, 50 U/mL DNAse I and 250 U/mL Collagenase I (Worthington Biochemical Corporation, Lakewood, NJ). The red blood cells in both the lungs and whole blood were lysed with RBC Lysis Buffer (eBiosceince). Single cell suspensions were stained according to standard surface stain protocols.

Antibodies against the following proteins were purchased from BD Bioscience (San Jose, CA): CD3e (500A2), CD4 (RM4-5), CD8a (53-6.7); BioLegend (San Diego, CA): CD3e (145-2C11), LAG-3 (C9B7W), PD-1 (CD279, 29F.1A12), Tim-3 (RMT3-23); or eBioscience (San Diego, CA): ICOS (7E.17G9), CD16/CD32 (93).

All samples were acquired on the BD LSRII or Fortessa and analyzed using FlowJo version 9.6.2 (TreeStar Inc., Ashland, OR).

All statistical analyses were performed using GraphPad Prism version 6.01 for Windows (GraphPad Software, La Jolla, CA).

### Example 3

### Increase in Tim-3 Expression with MVA-BN-HER2 Treatment

Tim-3 expression was measured by flow cytometry in mice after day 1 and 15 treatment with MVA-BN-HER2 (1E7 Inf.U., t.s.) as described in Example 2. Shown in Figure 1, the results demonstrate an increase in the percent of CD8 ⁺ T Cells expressing the TIM-3 after treatment with MVA-BN-HER2.

### Example 4

### MVA-BN-HER2 and anti-TIM-3 Treatment Reduces Tumor Growth

Mice were implanted i.d. with CT26-HER-2 tumors on day 1 and treated with MVA-BN-HER2 (1E7 Inf.U. t.s.) and anti-Tim-3 (200 µg, i.p.) on days 1 and 15 as described in Example 2. Shown in Figure 2, the results demonstrate that treatment with MVA-BN-HER2 in combination with anti-TIM-3 reduced tumor growth.

Shown in Figure 2, the results demonstrate that treatment with MVA-BN-HER2 and anti-TIM-3 reduced tumor growth and volume.

### Example 5

### MVA-BN-HER2 and anti-TIM-3 and anti-PD-1 Treatment Reduces Tumor Growth

Mice were implanted i.d. with CT26-HER-2 tumors on day 1 and treated with MVA-BN-HER2 (1E7 Inf.U. t.s.) and anti-Tim-3 and anti-PD-1 (200 µg each, i.p.) on days 1 and 15 as described in Example 2. Shown in Figure 3, the results demonstrate that treatment with MVA-BN-HER2 in combination with anti-TIM-3 and anti-PD-1 reduced tumor growth.

### Example 6

### MVA-BN-HER2 and anti-TIM-3 and LAG-3 Treatment Reduces Tumor Growth

Mice were implanted i.d. with CT26-HER-2 tumors on day 1 and treated with MVA-BN-HER2 (1E7 Inf.U. t.s.) and anti-Tim-3 and anti-LAG-3 (200 µg each, i.p.) on days 1 and 15 as described in Example 2. Shown in Figure 4, the results demonstrate that treatment with MVA-BN-HER2 in combination with anti-TIM-3 and anti-LAG-3 reduced tumor growth.

### Example 7

### MVA-BN-HER2 and anti-TIM-3 and anti-CTLA-4 Treatment Reduces Tumor Growth

Mice were implanted i.d. with CT26-HER-2 tumors on day 1 and treated with MVA-BN-HER2 (1E7 Inf.U. s.c..) and anti-Tim-3 (200 µg, i.p.) and anti-CTLA-4 (22 µg, i.p.) on days 1 and 15 as described in Example 2. Shown in Figure 5, the results demonstrate that treatment with MVA-BN-HER2 in combination with anti-TIM-3 and anti-LAG-3 reduced tumor growth. *** p<0.001, **** p<0.0001, Two-Way ANOVA.

### Example 8

### Induction of an anti-tumor response in mice treated with PROSTVAC and antibodies

Male BALB/c mice (6-8 weeks old, -20 g, Simonsen Laboratories, Gilroy CA) were implanted on day 1 with E6 cells (1.5×10⁵, i.d. in the back flank). Mice were treated on day 1 with PROSTVAC-V (2E7 Inf. U., s.c. at the tail base), and on days 8 and 15 with PROSTVAC-F (1E8 Inf. U., s.c. at the tail base). Mice were treated i.p. with anti-PD-1 and or anti-LAG-3 as described in Example 2.

### Example 9

### Induction of an anti-tumor response in mice treated with PROSTVAC and anti-PD-1

BALB/c mice were implanted i.d. with E6 tumors and treated with PROSTVAC and anti-PD-1 as described in Example 8. The results are shown in Figure 6.

### Example 10

### Induction of an anti-tumor response in mice treated with PROSTVAC and anti-LAG-3

BALB/c mice were implanted i.d. with E6 tumors and treated with PROSTVAC and anti-LAG-3 as described in Example 8. The results are shown in Figure 7.

### Example 11

### Induction of an anti-tumor response in mice treated with PROSTVAC and anti-PD-1 and anti-LAG-3

BALB/c mice were implanted i.d. with E6 tumors and treated with PROSTVAC and anti-PD-1 and anti-LAG-3 as described in Example 7. The results are shown in Figure 8.

### Example 12

### MVA-BN-CV301 with anti-CTLA-4 and anti-PD-1 Increases Overall Survival Rate

Female C57/BL6 mice (6-8 weeks old, ∼ 20 g, Simonsen Laboratories, Gilroy,CA) were implanted on day 1 i.v. with 1.0×10^6 MC38-MUC1 cells in 300 µL DPBS which forms tumors in the lungs. Mice were treated with MVA-BN-CV301 (4E5 Inf.U. subcutaneously, s.c. above the tail base) and treated with anti-CTLA-4 and anti-PD-1 (200 µg each) i.p. on days 4 and 18. MVA-BN-CV301

*Results.* Shown in Figure 9, the results demonstrate that MVA-BN-CV301 in combination with with anti-CTLA-4 and anti-PD-1 significantly increased overall survival rate of subjects as compared to treatment of cancers with either MVA-BN-CV301 or anti-CTLA-4 and anti-PD-1 alone.

### Example 13

### Heterologous prime boost amplifies PSA-specific T cell responses

BALB/c males (5/group) were treated every two weeks with: Buffer (Control), PROSTVAC-V (VVV) (2E6 Inf. U., s.c. at the tail base), PROSTVAC-F (FFF) (1E7 Inf. U., s.c. at the tail base), or received a PROSTVAC-V prime followed by 2 PROSTVAC-F boosts (VFF). Pooled splenocytes were assayed for PSA-specific responses by IFNγ ELISPOT as described in Mandl et al. Cancer Immunol. Immunother (2012), 61:19-29.

Results are shown in Figure 12. (A, B) and cytotoxic activity by flow cytometry (% CD107+ IFNγ+ CD8 T cells) (C). Anti-PSA IgG titers were determined by ELISA for each individual mouse (D). For ELISPOT, splenocytes were restimulated with CD4 or CD8 PSA-specific peptides or controls (controls not shown at indicated concentrations. Responses that were too numerous to count were displayed as 1000 spots/million cells. Statistical significance was determined by RM-ANOVA with Tukey post-test at 0.01 µM. ****P < 0.001 compared to control (A & B). To identify cytotoxic CD8+ T cells, splenocytes were restimulated overnight with a PSA CD8-specific peptide in the presence of anti-CD 107 antibody. Graphs show representative data of four independently performed experiments.

Shown in Figure 10, the heterologous prime-boost regimen with Vaccinia virus followed by one or more Fowlpoxvirus boost doses resulted in a much higher frequency of IFNγ-producing PSA-specific CD4 T cells (Figure 10A) and CD8 T cells (Figures 10B and 11A) compared to VVV or FFF homologous dosing regimens.

Moreover, PSA-specific T cells from VFF dosing were of higher avidity (Fig. 10A and 10B), as evidenced by higher frequencies of T cells responding at the lower 0.01 µM peptide concentrations in the ELISPOT. Importantly, the number of functionally active PSA-specific CD8 CTLs resulting from the VFF heterologous prime-boost regimen was 7 to 20 fold higher than those generated by either homologous dosing regimen (Figure 10C).

In contrast to the T cell responses, the heterologous prime-boost regimen did not improve PSA-specific antibody responses (Figure 10 D). These results indicate that heterologous VFF dosing generates CD4 and CD8 PSA-specific T cell responses of greater magnitude and higher quality as measured by higher avidity and increased CD8 CTL activity. As described herein, these contribute to improved anti-PSA specific anti-tumor responses following heterologous PROSTVAC-V/F dosing.

### Example 14

### Heterologous prime-boost improves the quality of PSA-specific T cell responses

BALB/c males (5/group) were treated as described in Example 40. Spleens were harvested 14 days after the last treatment, and pooled splenocytes were re-stimulated overnight with PSA OPL or controls (controls not shown). The cells were stained for intracellular IFNγ, TNFα, and IL-2 prior to flow cytometric analysis. (A) The pie charts are weighted in size to reflect the numbers of detected cells (total numbers of PSA-specific CD8 per million T cells are indicated below each chart). (B) Amount of IFNγ production on a per cell basis as measured by mean fluorescence intensity (MFI). Graphs show representative data of two independently performed experiments.

Shown in Figure 11, additional distinguishing features in the quality of the PSA-specific CD8 T cell response were observed when PSA-specific CD8 T cells were analyzed for the multicytokine-production of IFNγ, TNFα, and IL-2 by flow cytometry (Figure 11). Using cytokine expression, CD8 memory T cells have been classified as double-positive CD8 effector memory T cells (IFNγ+ TNFα+, TEM and as triple-positive CD8 central memory T cells (IFNγ+ TNFα+ IL-2+; TCM) *See, e.g.,* Nat Rev Immunol 2008, 8:247-258.

In addition to the increased magnitude of the CD8 T cell response (Fig. 10 and Fig. 11A), a pronounced shift in the quality of the CD8 T cell response was revealed by the higher proportion of double-positive TEM and triple-positive TCM (Fig.11A) as a result of the heterologous PROSTVAC-V/F regimen compared to homologous dosing regimen. Priming with a 5 fold higher PROSTVAC-V dose did not yield any additional benefit in the magnitude or the quality of the CD8 T cell response (data not shown). Further double-positive TEM and triple-positive TCM CD8 T cells produced higher levels of IFNγ on a per cell basis than single positive cells (Fig. 11B). This increased IFNγ production was observed in TEM and TCM CD8 T cells regardless of dosing regimen.

Additionally, shown in Figure 11, MVA-BN-HER2 induces tumor antigen specific T cells that produce IFNγ. It is contemplated by the present disclosure that virus induced TILs (tumor infiltrating lymphocytes) that secrete IFNγ may lead to increased PD-1 and/or PD-L1 on tumor cells; supporting blockade of this pathway in combination with virus treatment.

### Example 15

### Immune focusing of T cell response towards PSA

Mice were treated as described in Example 13. Pooled splenocytes were assayed for vaccinia virus (VV)-specific (A and C panels on left) or PSA-specific (A and C panels on right) cytotoxic activity by flow cytometry (% CD107+ IFNγ+ CD8 T cells) 14 days after the last treatment. Splenocytes were re-stimulated overnight with vaccinia E3L and F2L peptides or with PSA OPL in the presence of anti-CD 107 antibody. The following day, cells were stained intracellularly for IFNγ and with the surface markers CD127 and KLRG1. % antigen-specific cytotoxic SLEC and DPEC were determined by gating on (CD8 +CD127-KLRG1+) and (CD8+CD127+KLRG1+) cells, respectively. Graphs show representative data of two independently performed experiments. Results are shown in Figure 12.

The impact of heterologous PROSTVAC vaccinia virus Fowlpox/F dosing regimen compared to homologous dosing on the cytotoxic capabilities of vector-specific vs. PSA-specific effector T cell subsets was analyzed. Homologous VVV dosing generated a relatively high number of vaccinia-specific cytotoxic SLEC (-50%) and DPEC (-20%) (Figure. 12A and 12C), yet less than 10% of SLEC or DPEC cytotoxic CD8 T cells were PSA-specific. Conversely, 65% of SLEC and 30% of the highly active DPEC effector memory T cells were PSA-specific CTL following heterologous VFF dosing, while less than 10% constituted vaccinia-specific CTL (Figures 12A ,and 12C). Therefore, the heterologous PROSTVAC-V/F regimen resulted in a 100 fold improvement in the ratio of PSA-targeted to vaccinia-targeted SLEC and DPEC T cell responses (Figures 12B and 12D). Again, priming with 5 fold more PROSTVAC-V did not yield any additional benefit (data not shown).

Shown in Figure 12, additional distinguishing features in the quality of the PSA-specific CD8 T cell response were observed when PSA-specific CD8 T cells were analyzed for the multicytokine-production of IFNγ, TNFα, and IL-2 by flow cytometry (Figure 12). Using cytokine expression, CD8 memory T cells have been classified as double-positive CD8 effector memory T cells (IFNγ+ TNFα+, TEM and as triple-positive CD8 central memory T cells (IFNγ+ TNFα+ IL-2+; TCM) *See, e.g.,* Nat Rev Immunol 2008, 8:247-258.

In addition to the increased magnitude of the CD8 T cell response (Fig. 11 and Fig. 12A), a pronounced shift in the quality of the CD8 T cell response was revealed by the higher proportion of double-positive TEM and triple-positive TCM (Fig. 12A) as a result of the heterologous PROSTVAC-V/F regimen compared to homologous dosing regimen. Priming with a 5 fold higher PROSTVAC-V dose did not yield any additional benefit in the magnitude or the quality of the CD8 T cell response (data not shown). Further double-positive TEM and triple-positive TCM CD8 T cells produced higher levels of IFNγ on a per cell basis than single positive cells (Fig. 12B). This increased IFNγ production was observed in TEM and TCM CD8 T cells regardless of dosing regimen.

### Example 16

### Combination therapy with CTLA-4 after immune focusing

BALB/c males (5/group) are treated every two weeks with: Buffer (Control), PROSTVAC-V prime followed by 2 PROSTVAC-F boosts (VFF) as described in example 40. Mice are treated i.p. with anti-CTLA-4 (60 µg) on days 1, 15 and 29 (A), or on days 15 and 29 (B), or on day s16 and 30 (C) or on days 17 and 31.(D). PSA specific T cell responses are analyzed as described in examples 13, 14, and 15.

## Claims

1. A combination for use in treating a human cancer patient comprising: (a) a recombinant poxvirus encoding a polypeptide comprising at least one tumor-associated antigen (TAA); and (b) a TIM-3 antagonist that is an antibody, an antisense RNA or a siRNA.

2. The combination for use of claim 1, wherein the TIM-3 antagonist is an anti-TIM-3 antibody.

3. The combination for use of claim 1 or 2, further comprising (c) an antagonist of an immune checkpoint molecule selected from the group consisting of: PD-1, LAG-3, CTLA-4 and combinations thereof, wherein the antagonist is an antibody, an antisense RNA or a siRNA.

4. The combination for use of claim 3, wherein the PD-1 antagonist comprises an anti-PD-1 antibody.

5. The combination for use of claim 3, wherein the CTLA-4 antagonist is an antibody that inhibits CTLA-4 function.

6. The combination for use of any of claims 1-5, wherein the poxvirus is selected from an orthopoxvirus and an avipoxvirus.

7. The combination for use of claim 6, wherein the orthopoxvirus is a vaccinia virus, preferably a modified vaccinia Ankara (MVA) virus, more preferably MVA-BN.

8. The combination for use of claim 6, wherein the avipoxvirus is a fowlpox virus.

9. The combination for use of claims 1-8, wherein the at least one TAA is selected from the group consisting of: carcinoembryonic (CEA), mucin 1 cell surface associated (MUC-1), prostatic acid phosphatase (PAP), prostate specific antigen (PSA), human epidermal growth factor receptor 2 (HER-2), survivin, tyrosine related protein 1 (tyrp1), tyrosine related protein 1 (tyrp2), Brachyury antigen and combinations thereof.

10. The combination for use of claims 1-9, wherein the at least one TAA is CEA and MUC-1.

11. The combination for use of claims 1-10, wherein the cancer treatment is directed against breast cancer, lung cancer, head and neck cancer, thyroid, melanoma, gastric cancer, bladder cancer, kidney cancer, liver cancer, melanoma, pancreatic cancer, prostate cancer, ovarian cancer, or colorectal cancer.

12. The combination for use of claims 1-11, wherein the combination is administered as part of a homologous or heterologous prime-boost regimen;
wherein the homologous prime-boost regimen comprises a first prime dose of the recombinant poxvirus in combination with the immune checkpoint antagonist and one or more subsequent boost doses of a same recombinant poxvirus in combination with the immune checkpoint antagonist; and
wherein the heterologous prime-boost regimen comprises a first prime dose of the recombinant poxvirus in combination with the immune checkpoint antagonist and one or more subsequent boost doses of a different recombinant poxvirus in combination with the immune checkpoint antagonist.

## Patentansprüche

1. Kombination zur Verwendung bei der Behandlung eines menschlichen Krebspatienten, umfassend: (a) ein rekombinantes Pockenvirus, das ein wenigstens ein tumorassoziiertes Antigen (TAA) umfassendes Polypeptid codiert; und (b) einen TIM-3-Antagonisten, bei dem es sich um einen Antikörper, eine Antisense-RNA oder eine siRNA handelt.

2. Kombination zur Verwendung nach Anspruch 1, wobei es sich bei dem TIM-3-Antagonisten um einen Anti-TIM-3-Antikörper handelt.

3. Kombination zur Verwendung nach Anspruch 1 oder 2, ferner umfassend (c) einen Antagonisten eines Immuncheckpoint-Moleküls ausgewählt aus der Gruppe bestehend aus: PD-1, LAG-3, CTLA-4 und Kombinationen davon, wobei es sich bei dem Antagonisten um einen Antikörper, eine Antisense-RNA oder eine siRNA handelt.

4. Kombination zur Verwendung nach Anspruch 3, wobei der PD-1-Antagonist einen Anti-PD-1-Antikörper umfasst.

5. Kombination zur Verwendung nach Anspruch 3, wobei es sich bei dem CTLA-4-Antagonisten um einen Antikörper handelt, der CTLA-4-Funktion hemmt.

6. Kombination zur Verwendung nach einem der Ansprüche 1-5, wobei das Pockenvirus aus einem Orthopoxvirus und einem Avipoxvirus ausgewählt ist.

7. Kombination zur Verwendung nach Anspruch 6, wobei es sich bei dem Orthopoxvirus um ein Vacciniavirus, bevorzugt ein Modifiziertes-Vaccinia-Ankara(MVA)-Virus, stärker bevorzugt MVA-BN handelt.

8. Kombination zur Verwendung nach Anspruch 6, wobei es sich bei dem Avipoxvirus um ein Hühnerpockenvirus handelt.

9. Kombination zur Verwendung nach Anspruch 1-8, wobei das wenigstens eine TAA ausgewählt ist aus der Gruppe bestehend aus: CEA (Carcinoembryonic), MUC-1 (Mucin 1 Cell Surface Associated), PAP (Prostatic Acid Phosphatase), PSA (Prostate Specific Antigen), HER-2 (Human Epidermal Growth Factor Receptor 2), Survivin, tyrp1 (Tyrosine Related Protein 1), tyrp2 (Tyrosine Related Protein 1), Brachyury-Antigen und Kombinationen davon.

10. Kombination zur Verwendung nach Anspruch 1-9, wobei es sich bei dem wenigstens einen TAA um CEA und MUC-1 handelt.

11. Kombination zur Verwendung nach Anspruch 1-10, wobei die Krebsbehandlung gegen Brustkrebs, Lungenkrebs, Krebs im Kopf- und Halsbereich, Schilddrüse, Melanom, Magenkrebs, Blasenkrebs, Nierenkrebs, Leberkrebs, Melanom, Bauchspeicheldrüsenkrebs, Prostatakrebs, Ovarialkarzinom oder Kolorektalkarzinom gerichtet ist.

12. Kombination zur Verwendung nach Anspruch 1-11, wobei die Kombination als Teil eines homologen oder heterologen Prime-Boost-Schemas verabreicht wird;
wobei das homologe Prime-Boost-Schema eine erste Prime-Dosis des rekombinanten Pockenvirus in Kombination mit dem Immuncheckpoint-Antagonisten und eine oder mehrere nachfolgende Boost-Dosen eines identischen rekombinanten Pockenvirus in Kombination mit dem Immuncheckpoint-Antagonisten umfasst; und
wobei das heterologe Prime-Boost-Schema eine erste Prime-Dosis des rekombinanten Pockenvirus in Kombination mit dem Immuncheckpoint-Antagonisten und eine oder mehrere nachfolgende Boost-Dosen eines unterschiedlichen rekombinanten Pockenvirus in Kombination mit dem Immuncheckpoint-Antagonisten umfasst.

## Revendications

1. Combinaison pour utilisation dans le traitement d'un patient cancéreux humain comprenant : (a) un poxvirus recombinant codant pour un polypeptide comprenant au moins un antigène associé à une tumeur (TAA) ; et (b) un antagoniste de TIM-3 qui est un anticorps, un ARN antisens ou un pARNi.

2. Combinaison pour utilisation selon la revendication 1, dans laquelle l'antagoniste de TIM-3 est un anticorps anti-TIM-3.

3. Combinaison pour utilisation selon la revendication 1 ou 2, comprenant en outre (c) un antagoniste d'une molécule de point de contrôle immunitaire choisie dans le groupe constitué de : PD-1, LAG-3, CTLA-4 et des combinaisons de ceux-ci, dans laquelle l'antagoniste est un anticorps, un ARN antisens ou un pARNi.

4. Combinaison pour utilisation selon la revendication 3, dans laquelle l'antagoniste de PD-1 comprend un anticorps anti-PD-1.

5. Combinaison pour utilisation selon la revendication 3, dans laquelle l'antagoniste de CTLA-4 est un anticorps qui inhibe la fonction de CTLA-4.

6. Combinaison pour utilisation selon l'une quelconque des revendications 1 à 5, dans laquelle le poxvirus est choisi parmi un orthopoxvirus et un avipoxvirus.

7. Combinaison pour utilisation selon la revendication 6, dans laquelle l'orthopoxvirus est un virus de la vaccine, de préférence un virus de la vaccine Ankara modifié (MVA), plus préférablement MVA-BN.

8. Combinaison pour utilisation selon la revendication 6, dans laquelle l'avipoxvirus est un virus de la variole aviaire.

9. Combinaison pour utilisation selon les revendications 1 à 8, dans laquelle l'au moins un TAA est choisi dans le groupe constitué des : antigène carcino-embryonnaire (CEA), antigène associé à la surface cellulaire mucine 1 (MUC-1), phosphatase acide prostatique (PAP), antigène spécifique de la prostate (PSA), récepteur de facteur de croissance épidermique humain 2 (HER-2), survivine, protéine associée à la tyrosine 1 (tyrp1), protéine associée à la tyrosine 2 (tyrp2), antigène de brachyurie et des combinaisons de ceux-ci.

10. Combinaison pour utilisation selon les revendications 1 à 9, dans laquelle l'au moins un TAA est CEA et MUC-1.

11. Combinaison pour utilisation selon les revendications 1 à 10, le traitement du cancer étant dirigé contre un cancer du sein, un cancer du poumon, un cancer de la tête et du cou, un cancer de la thyroïde, un mélanome, un cancer de l'estomac, un cancer de la vessie, un cancer rénal, un cancer du foie, un mélanome, un cancer du pancréas, un cancer de la prostate, un cancer des ovaires ou un cancer colorectal.

12. Combinaison pour utilisation selon les revendications 1 à 11, la combinaison étant administrée dans le cadre d'un protocole de primo-vaccination et rappel homologue ou hétérologue ;
où le protocole de primo-vaccination et rappel homologue comprend une première dose de primo-vaccination du poxvirus recombinant en combinaison avec l'antagoniste de point de contrôle immunitaire et une ou plusieurs doses de rappel consécutives d'un même poxvirus recombinant en combinaison avec l'antagoniste de point de contrôle immunitaire ; et
où le protocole de primo-vaccination et rappel hétérologue comprend une première dose de primo-vaccination du poxvirus recombinant en combinaison avec l'antagoniste de point de contrôle immunitaire et une ou plusieurs doses de rappel consécutives d'un poxvirus recombinant différent en combinaison avec l'antagoniste de point de contrôle immunitaire.
